# EUROPEAN PATENT APPLICATION

(11) **EP 4 474 391 A2**
(43) Date of publication of application: **11.12.2024**
(21) Application number: 24194391.9
(22) Date of filing: 14.05.2019
(51) Int. Cl.: C07K 14/705

(54) **SUBSETS OF HUMAN NATURAL KILLER CELLS WITH ENHANCED ANTIBODY-DIRECTED IMMUNE RESPONSES**

(30) Priority: 14.05.2018 US 201862671358 P
(62) Divisional of application: 19803497.7
(71) Applicant: Indapta Therapeutics, Inc., Houston, TX 77006 (US)
(72) Inventor: DOS SANTOS, Gary, Seattle, 98107 (US); DIPIERRO, Guy, Houston, 77006 (US)
(74) Representative: J A Kemp LLP

(57) **Abstract**

Provided herein are compositions containing specialized subsets of natural killer (NK) cells with enhanced antibody-directed immune responses. The compositions are useful for treating diseases and conditions such as cancer, including in combination with an antibody capable of binding to disease-associated tissues or cells, such as tumor cells or infected cells.

## Description

### Cross-Reference to Related Applications

This application claims priority from U.S. provisional application No. 62/671,358, filed May 14, 2018, entitled "SUBSETS OF HUMAN NATURAL KILLER CELLS WITH ENHANCED ANTIBODY DIRECTED IMMUNE RESPONSES," the contents of which are incorporated by reference in their entirety.

### Incorporation by Reference of Sequence Listing

The present application is being filed along with a Sequence Listing in electronic format. The Sequence Listing is provided as a file entitled 776032000340SeqList.txt, created May 14, 2019, which is 7.04 kilobytes in size. The information in the electronic format of the Sequence Listing is incorporated by reference in its entirety.

### Field

The present disclosure provides compositions containing specialized subsets of natural killer (NK) cells with enhanced antibody-directed immune responses. The compositions are useful for treating diseases and conditions such as cancer, including in combination with an antibody capable of binding to disease-associated tissues or cells, such as tumor cells or infected cells.

### Background

Antibody-based therapy has become frequently used for treating cancers and other disease indications. Responses to antibody therapy has typically focused on the direct inhibitory effects of these antibodies on the tumor cells (e.g. inhibition of growth factor receptors and the subsequent induction of apoptosis), but the in vivo effects of these antibodies may be more complex and may involve the host immune system. Natural killer (NK) cells are immune effector cells that mediate antibody-dependent cellular cytotoxicity when the Fc receptor (CD16; FcyRIII) binds to the Fc portion of antibodies bound to an antigen-bearing cell. Improved therapies are needed to improve responses to antibody therapy. Provided herein are embodiments that meet such needs.

### Summary

Provided herein are specialized subsets of NK cells that exhibit enhanced antibody-directed immune responses. In some embodiments, compositions enriched for the NK cell subsets are provided. In some embodiments, the compositions can be administered to a subject for treating a disease or condition in combination with an antibody or other Fc protein, such as an antibody or Fc protein for treating the disease or condition or that binds or recognizes an antigen or protein associated with the disease or condition. In some embodiments, the provided methods achieve an improved response compared to methods in which conventional NK cells are administered or compared to methods in which the antibody or Fc alone is administered. In some embodiments, the increased response is due to increased affinity of the Fc fragment of IgG antibodies by the specialized NK cells, which can result in enhanced antibody dependent cell mediated cytotoxicity (ADCC) and hence increased killing of cells targeted by the antibody or protein, e.g. tumor cells. In some embodiments, the specialized NK cells are surface positive for CD16 having the 158V+ polymorphism and/or are deficient in expression of the FcRy chain.

Provided herein is a composition comprising a natural killer (NK) cell subset surface positive for CD16 158V+, wherein at least about 50%, at least about 60%, at least about 70%, at least about 80%, or at least about 90% of the cells in the composition comprise the CD16 158V+ NK cell subset. In some embodiments, the CD16 158V+ NK cell subset does not express FcRy chain (CD16 158V+/g-), is surface positive for a KIR and/or is surface negative or low for Nkp30 and/or Nkp46.

Provided herein is a composition comprising a natural killer (NK) cell subset surface positive for CD16 158V+ and deficient in expression of FcRy chain (g-) (CD16 158V+/g-), wherein at least about 50%, at least about 60%, at least about 70%, at least about 80%, or at least about 90% of the cells in the composition comprise the CD16 158V+/g- NK cell subset.

In some of any of the provided embodiments, CD16 158V+ is a surface receptor that comprises the sequence set forth in SEQ ID NO:11 or a sequence that exhibits at least 85%, 90% or 95% sequence identity to SEQ ID NO:11 and comprises the 158V+ polymorphism.

In some of any of the provided embodiments, the NK cell subset of the composition is surface positive for a KIR, optionally KIR2DL2/3. In some of any of the provided embodiments, the NK cell subset of the composition is negative or low for surface expression of Nkp30 and/or Nkp46. In some of any of the provided embodiments, the NK cell subset of the composition is surface positive for a KIR, optionally KIR2DL2/3 and is negative or low for surface expression of Nkp30 and/or Nkp46.

In some of any of the provided embodiments, the NK cell subset of the composition is surface positive for NKG2C.

In some of any of the provide embodiments, the composition comprises cells in which the NK cell subset is present from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells. In some embodiments, the volume of the composition is at least or at least about 10 mL, 50 mL, 100 mL, 200 mL, 300 mL, 400 mL or 500 mL and/or the composition comprises a concentration of the NK cell subset that is between 1 × 10⁵ and 1 × 10⁸ cells/mL.

In some of any such embodiments, the composition contains a pharmaceutically acceptable carrier. In some embodiments, the composition contains a cyroprotectant.

In some of any such embodiments, the NK cell subset present in the composition includes primary NK cells obtained from a subject. In some embodiments, the subject is human.

Provided herein is a method for enriching NK cells, the method comprising: (a) isolating NK cells or a subset thereof from a subject identified as having the CD16 158V+ NK cell genotype; and (b) culturing the isolated NK cells or subset thereof under conditions to expand the cells, thereby enriching NK cells or the subset thereof from the subject. In some embodiments, prior to step (a), the method includes screening or identifying subjects for the presence of the CD16 158V+ NK cell genotype.

In some of any such embodiments, the NK cells or subset thereof are isolated from a population of cells comprising lymphocytes. In some embodiments, the population of cells comprises peripheral blood mononuclear cells (PBMC) sample, an apheresis product, or a leukapheresis product.

In some of any such embodiments, the method includes isolating an NK cell subset from the subject, in which the NK cell subset comprises or is enriched for cells that do not express FcRy chain (CD16 158V+/g-). In some embodiments, the NK cell subset is surface positive for a KIR, optionally KIR2DL2/3. In some embodiments, the NK cell subset is negative or low for surface expression of Nkp30 and/or Nkp46. In some embodiments, the NK cell subset is surface positive for a KIR, optionally KIR2DL2/3 and is negative or low for surface expression of Nkp30 and/or Nkp46. In some embodiments, the NK cells or subset thereof is surface positive for NKG2C.

In some of any of such embodiments, the method comprises administering IL-12, IL-15, IL-18, IL-2 and/or CCL5 to the subject prior to isolating the NK cells or subset thereof.

In some of any such embodiments, the cells are cultured in the presence of feeder cells or in the presence of one or more cytokine, optionally IL-2, IL-15 and/or IL-7. In some embodiments, the NK cells are cultured for a period of time to achieve expansion of the cells by at least 5-fold, 10-fold, 20-fold, 30-fold, 40-fold, 50-fold, 100-fold, 200-fold, 300-fold, 400-fold, 500-fold or more compared to the number of isolated NK cells prior to the culturing. In some embodiments, the isolated NK cells are cultured for 7 to 28 days, optionally about or at least about 14 days, 15 days, 16 days, 17 days, 18 days, 19 days, 20 days, or 21 days. In some embodiments, the isolated cells are cultured to achieve a therapeutically effective amount of the NK cells or subset thereof. In some embodiments, the isolated cells are cultured to achieve a number of enriched NK cells or subset thereof from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells.

In some of any such embodiments, the method further comprises HLA typing the isolated or enriched cells. In some embodiments, the method further comprises cryopreserving the cells and/or formulating the cells in the presence of a cryoprotectant. In some embodiments, the resulting enriched cells are administered to a subject for treating a disease or disorder. In some of any such embodiments, the subject is human. In some embodiments, the subject is healthy.

Also provided is a composition containing enriched NK cells or a subset thereof produced by any of the above methods. In some embodiments, the composition contains a pharmaceutically acceptable excipient. In some embodiments, the composition contains a cyroprotectant.

In some of any such embodiments, the composition is sterile.

Provided herein is a kit containing a composition of any of the above embodiments and an additional agent for treatment of a disease. In some embodiments, the kit further contains instructions for administering the composition and additional agent for treating a disease or condition.

Provided herein is a kit containing the composition of any of the provided embodiments and instructions for administering the composition in a combination therapy with an additional agent for treatment of a disease.

In some of any of such embodiments, the additional agent is an antibody or an Fc-fusion protein. In some embodiments, the antibody recognizes or specifically binds a tumor associated antigen. In some embodiments, the antibody recognizes or binds CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin. In some embodiments, the antibody is a full length antibody and/or comprises an Fc domain.

Provided herein is a method of treating a disease or condition by administering the composition of any of the above embodiments to an individual or subject in need thereof. In some embodiments, the method includes administering from or from about 1 × 10⁸ to 1 × 10¹⁰ cells/m² to the individual or administering from or from about 1 × 10⁶ to 1 × 10¹⁰ NK cells/kg.

In some of any such embodiments, the method further includes administering an additional agent. In some embodiments, the additional agent is an antibody or an Fc-fusion protein. In some embodiments, the antibody recognizes a tumor associated antigen. In some embodiments, the antibody recognizes or specifically binds CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin. In some embodiments, the antibody comprises an Fc domain and/or is a full-length antibody.

In some of any such embodiments, the additional agent and the composition are administered sequentially. In some embodiments, the additional agent is administered prior to administration of the composition. In some embodiments, the additional agent and the composition are administered simultaneously.

In some of any such embodiments, the disease or condition is selected from the group consisting of an inflammatory condition, an infection, and cancer. In some embodiments, the infection is a viral infection or a bacterial infection. In some embodiments, the cancer is leukemia or lymphoma. In some embodiments, the cancer comprises a solid tumor.

In some of any such embodiments, the individual is a human. In some of any such embodiments, the administered NK cells in the composition are autologous to the subject. In some of any such embodiments, the administered NK cells in the composition are allogenic to the individual. In some embodiments, the cells are administered to a subject of the same HLA type. In some embodiments, the cells from an HLA-matched donor are administered to a subject.

In some embodiments, the method includes thawing the cells prior to administering the cells to a subject.

### Brief Description of the Drawings

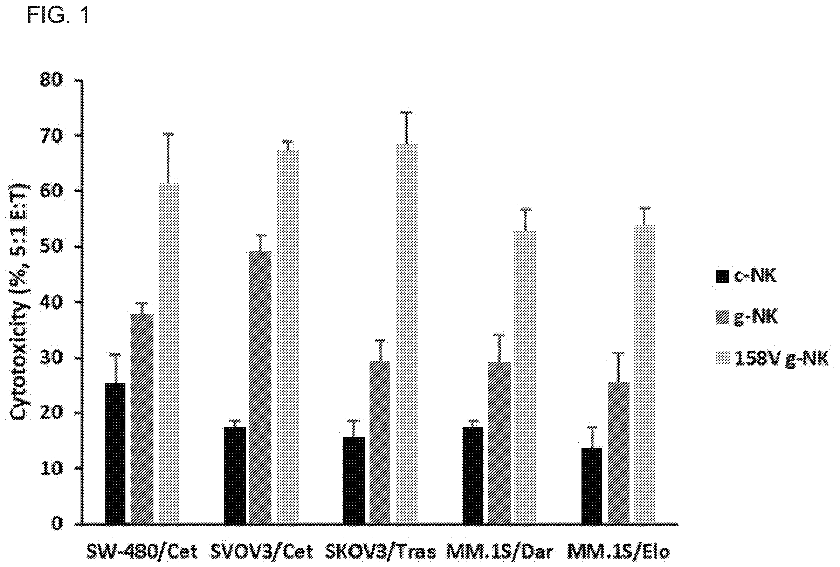

FIG. 1 shows ADCC activity of g-NK, conventional NK cells, and 158V g-NK against SW-480/cetuximab, SKOV3/cetuximab, SKOV3/trastuzumab, MM.1S/daratumumab, and MM.1S/elotuzumab at a 5:1 E:T ratio. Values are mean ± standard error.

### Detailed Description

Provided herein are compositions containing subsets of human Natural Killer (NK) cells with enhanced antibody-directed immune responses, including compositions enriched for such subsets. In some embodiments, the provided compositions contain or are enriched for NK cells bearing specific genetic polymorphisms of the CD16 (FcyRIII) gene at the 158 amino acid position, where a valine (V) has been substituted for a phenyalanine (F) (referred to as 158V+). In some embodiments, the provided compositions contain or are enriched for 158V+ and for FcRy deficient NK cells (referred to as g-NK), thereby providing a composition containing or enriched for CD16 158V+/g- NK cells. Also provided are methods for administering the provided compositions to a subject in combination with an antibody for treating a disease or condition.

In some embodiments, the provided compositions comprise human NK cells in which greater than or greater than about 40%, 50%, 60%, 70%, 80%, 90% or more of the NK cells in the composition or of the total cells of the composition are CD16 158V+ NK cells or CD16 158V+/g- NK cells.

Natural killer (NK) cells are innate lymphocytes important for mediating anti-viral and anti-cancer immunity through cytokine and chemokine secretion, and through the release of cytotoxic granules (Vivier et al. Science 331(6013):44-49 (2011); Caligiuri, Blood 112(3):461-469 (2008); Roda et al., Cancer Res. 66(1):517-526 (2006)). NK cells are effector cells that comprise the third largest population of lymphocytes and are important for host immuno-surveillance against tumor and pathogen-infected cells. However, unlike T and B lymphocytes, NK cells are thought to have only a limited capacity for target recognition using germline-encoded activation receptors (Bottino et al., Curr Top Microbiol Immunol. 298:175-182 (2006); Stewart et al., Curr Top Microbiol Immunol. 298:1-21 (2006)).

Activation of NK cells can occur through the direct binding of NK cell receptors to ligands on the target cell, as seen with direct tumor cell killing, or through the crosslinking of the Fc receptor (CD16; also known as CD16a or FcyRIIIa) by binding to the Fc portion of antibodies bound to an antigen-bearing cell. Upon activation, NK cells produce cytokines and chemokines abundantly and at the same time exhibit potent cytolytic activity. NK cells are capable of killing tumor cells via antibody dependent cell mediated cytotoxicity (ADCC). In some cases, ADCC is triggered when receptors on the NK cell surface (such as CD16) recognize IgGl or IgG3 antibodies bound to the surface of a cell. This triggers release of cytoplasmic granules containing perforin and granzymes, leading to target cell death. Because NK cells express the activating Fc receptor CD16, which recognizes IgG-coated target cells, target recognition is broadened (Ravetch & Bolland, Annu Rev Immunol. 19:275-290 (2001); Lanier Nat. Immunol. 9(5):495-502 (2008); Bryceson & Long, Curr Opin Immunol. 20(3):344-352 (2008)). ADCC and antibody-dependent cytokine/chemokine production are primarily mediated by NK cells.

In addition to CD16 expressed on NK cells that is involved in antibody-dependent responses (such as NK cell-mediated ADCC), CD16 also exists as glycosylphosphatidylinositol-anchored form (also known as FcyRIIIB or CD16B). It is understood that reference to CD16 herein is with reference to the CD16a form that is expressed on NK cells and is not meant to refer to the glycosylphosphatidylinositol-anchored form. The CD16 receptor is able to associate with adaptors, the ζ chain of the TCR-CD3 complex (CD3ζ) and/or the FcRy chain, to transduce signals through immunoreceptor tyrosine-based activation motifs (ITAMs). In some aspects, CD16 engagement (CD16 crosslinking) initiates NK cell responses via intracellular signals that are generated through one, or both, of the CD16-associated adaptor chains, FcRy or CD3ζ. Triggering of CD16 leads to phosphorylation of the y or ζ chain, which in turn recruits tyrosine kinases, syk and ZAP-70, initiating a cascade of signal transduction leading to rapid and potent effector functions. The most well-known effector function is the release of cytoplasmic granules carrying toxic proteins to kill nearby target cells through the process of antibody-dependent cellular cytotoxicity. CD16 crosslinking also results in the production of cytokines and chemokines that, in turn, activate and orchestrate a series of immune responses.

This release of cytokines and chemokines can play a role in the anti-cancer activity of NK cells in vivo. NK cells also have small granules in their cytoplasm containing perforin and proteases (granzymes). Upon release from the NK cell, perforin forms pores in the cell membrane of targeted cells through which the granzymes and associated molecules can enter, inducing apoptosis. The fact that NK cells induce apoptosis rather than necrosis of target cells is significant-necrosis of a virus-infected cell would release the virions, whereas apoptosis leads to destruction of the virus inside the cells.

The majority of humans express CD16 which has a relatively low affinity for IgGl antibodies. However, it has been found that NK cells from individuals bearing a single nucleotide polymorphism (SNP rs396991) in the CD16 gene, nucleotide 526 [thymidine (T) → guanine (G)] resulting in an amino acid (aa) substitution of valine (V) for phenylalanine (F) at position 158 (F158V, also called 158V+ herein) in the mature (processed) form of the protein, is associated with substantially higher affinity for IgGl antibodies and have the ability to mount more robust NK cell-mediated ADCC responses (Mellor et al. (2013) Journal of Hematology & Oncology, 6:1; Musolino et al. (2008) Journal of Clinical Oncology, 26:1789-1796 and Hatjiharissi et al. (2007) Blood, 110:2561-2564).

It also has been found that subsets of FcRy chain deficient NK cells (referred to as g-NK) are also able to mediate robust ADCC responses (see e.g. published Patent Appl. No. US2013/0295044). The mechanism for increased responses may be due to changes in epigenetic modification that influence the expression of the FcRy. The g-NK cells express the signaling adaptor ζ chain abundantly, but are deficient in the expression of the signaling adaptor y chain. Compared to conventional NK cells, these γ-deficient g-NK cells exhibit dramatically enhanced activity when activated by antibodies. For example, the g-NK cells can be activated by antibody-mediated crosslinking of CD16 or by antibody-coated tumor cells. In some aspects, the g-NK cells produce greater amounts of cytokines (e.g. IFN-y or TNF-α) and chemokines (e.g. MIP-1α, MIP-1β, and RANTES) and/or display higher degranulation responses than conventional NK cells expressing the y chain. The g-NK cells provide high expression of Granzyme B, a component of natural killer cell cytotoxic machinery. Moreover, the g-NK cells have a prolonged lifespan, compared to conventional NK cells, and their presence is maintained long-term. In some embodiments, g-NK cells are functionally and phenotypically stable.

In some embodiments, CD16 158V+ NK cells and CD16 158V+/g- NK cells are more effective in eliciting ADCC responses than conventional NK cells, e.g. NK cells that do not contain the 158V+ polymorphism and/or that are not deficient in the y chain. In some cases, ADCC is a mechanism of action of therapeutic antibodies, including anti-cancer antibodies. In some aspects, cell therapy by administering NK cells can be used in concert with antibodies for therapeutic and related purposes. Provided herein are methods involving combined administration of a composition enriched for CD16 158V+ NK cells or CD16 158V+/g- NK cells as provided and an antibody, e.g. an anti-cancer antibody. In some embodiments, antibody-directed targeting of CD16 158V+ NK cells or CD16 158V+/g- NK cells leads to improved outcomes for patients due to the improved affinity, cytotoxic and/or cytokine-mediated effect functions of the CD16 158V+ NK cells or CD16 158V+/g- NK cell subsets.

In some embodiments, the g-NK cells produce significantly greater amounts of a cytokine than natural killer cells that do express FcRy. In another embodiment, the cytokine is interferon-gamma (IFN-y), tumor necrosis factor-α (TNF-α), or a combination thereof. In one embodiment, the g-NK cells produce significantly greater amounts of a chemokine. In one embodiment, the chemokine is MIP-1α, MIP-1β or a combination thereof. In another embodiment, the g-NK cells produce the cytokine or the chemokine upon stimulation through the Fc receptor CD16.

All references cited herein, including patent applications, patent publications, and scientific literature and databases, are herein incorporated by reference in their entirety for all purposes to the same extent as if each individual reference were specifically and individually indicated to be incorporated by reference.

For clarity of disclosure, and not by way of limitation, the detailed description is divided into the subsections that follow. The section headings used herein are for organizational purposes only and are not to be construed as limiting the subject matter described.

### I. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used in this specification and the appended claims, the singular forms "a", "an" and "the" include plural referents unless the content clearly dictates otherwise. Thus, for example, reference to "a molecule" optionally includes a combination of two or more such molecules, and the like.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of" aspects and embodiments.

As used herein, "optional" or "optionally" means that the subsequently described event or circumstance does or does not occur, and that the description includes instances where said event or circumstance occurs and instances where it does not. For example, an optionally substituted group means that the group is unsubstituted or is substituted.

As used herein, "antibody" refers to immunoglobulins and immunoglobulin fragments, whether natural or partially or wholly synthetically, such as recombinantly, produced, including any fragment thereof containing at least a portion of the variable heavy chain and/or light chain region of the immunoglobulin molecule that is sufficient to form an antigen binding site and, when assembled, to specifically bind antigen. Hence, an antibody includes any protein having a binding domain that is homologous or substantially homologous to an immunoglobulin antigen-binding domain (antibody combining site). Typically, antibodies minimally include all or at least a portion of the variable heavy (V_{H}) chain and/or the variable light (V_{L}) chain. In general, the pairing of a V_{H} and V_{L} together form the antigen-binding site, although, in some cases, a single V_{H} or V_{L} domain is sufficient for antigen-binding. The antibody also can include all or a portion of the constant region. Reference to an antibody herein includes full-length antibody and antigen-binding fragments. The term "immunoglobulin" (Ig) is used interchangeably with "antibody" herein.

The terms "full-length antibody," "intact antibody" or "whole antibody" are used interchangeably to refer to an antibody in its substantially intact form, as opposed to an antibody fragment. A full-length antibody is an antibody typically having two full-length heavy chains (e.g., VH-CH1-CH2-CH3 or VH-CH1-CH2-CH3-CH4) and two full-length light chains (VL-CL) and hinge regions, such as antibodies produced from mammalian species (e.g. human, mouse, rat, rabbit, non-human primate, etc.) by antibody secreting B cells and antibodies with the same domains that are produced synthetically. Specifically whole antibodies include those with heavy and light chains including an Fc region. The constant domains may be native sequence constant domains (*e.g.,* human native sequence constant domains) or amino acid sequence variants thereof. In some cases, the intact antibody may have one or more effector functions.

An "antibody fragment" comprises a portion of an intact antibody, the antigen binding and/or the variable region of the intact antibody. Antibody fragments, include, but are not limited to, Fab fragments, Fab' fragments, F(ab')₂ fragments, Fv fragments, disulfide-linked Fvs (dsFv), Fd fragments, Fd' fragments; diabodies; linear antibodies (see U.S. Pat. No. 5,641,870, Example 2; Zapata et al., Protein Eng. 8(10): 1057-1062 [1995]); single-chain antibody molecules, including single-chain Fvs (scFv) or single-chain Fabs (scFab); antigen-binding fragments of any of the above and multispecific antibodies from from antibody fragments. For purposes herein, an antibody fragment typically includes one that is sufficient to engage or crosslink CD16 on the surface of an NK cell.

The term "autologous" refers to cells or tissues originating within or taken from an individual's own tissues. For example, in an autologous transfer or transplantation of NK cells, the donor and recipient are the same person.

The term "allogeneic" refers to cells or tissues that belong to or are obtained from the same species but that are genetically different, and which, in some cases, are therefore immunologically incompatible. Typically, the term "allogeneic" is used to define cells that are transplanted from a donor to a recipient of the same species.

The term "expression" refers to the process by which a polynucleotide is transcribed from a DNA template (such as into an mRNA or other RNA transcript) and/or the process by which a transcribed mRNA is subsequently translated into peptide, polypeptides or proteins. Transcripts and encoded polypeptides may be collectively referred to as "gene product." If the polynucleotide is derived from genomic DNA, expression may include splicing of the mRNA in a eukaryotic cell.

The term "heterologous" with reference to a protein or nucleic acid refers to a protein or nucleic acid originating from a different genetic source. For example, a protein or nucleic acid that is heterologous to a cell originates from an organism or individual other than the cell in which it is expressed.

As used herein, the term "introducing" encompasses a variety of methods of introducing DNA into a cell, either in vitro or in vivo, such methods including transformation, transduction, transfection (e.g. electroporation), and infection. Vectors are useful for introducing DNA encoding molecules into cells. Possible vectors include plasmid vectors and viral vectors. Viral vectors include retroviral vectors, lentiviral vectors, or other vectors such as adenoviral vectors or adeno-associated vectors.

The term "composition" refers to any mixture of two or more products, substances, or compounds, including cells or antibodies. It may be a solution, a suspension, liquid, powder, a paste, aqueous, non-aqueous or any combination thereof. The preparation is generally in such form as to permit the biological activity of the active ingredient (e.g. antibody) to be effective.

The term "enriched" with reference to a cell composition refers to a composition in which there is an increase in the number or percentage of the cell type or population as compared to the number or percentage of the cell type in a starting composition of the same volume, such as a starting composition directly obtained or isolated from a subject. The term does not require complete removal of other cells, cell type, or populations from the composition and does not require that the cells so enriched be present at or even near 100 % in the enriched composition.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, combination refers to any association between or among two or more items. The combination can be two or more separate items, such as two compositions or two collections, can be a mixture thereof, such as a single mixture of the two or more items, or any variation thereof. The elements of a combination are generally functionally associated or related.

As used herein, a kit is a packaged combination that optionally includes other elements, such as additional agents and instructions for use of the combination or elements thereof, for a purpose including, but not limited to, therapeutic uses.

As used herein, the term "treatment" or "treating" refers to clinical intervention designed to alter the natural course of the individual or cell being treated during the course of clinical pathology. Desirable effects of treatment include decreasing the rate of disease progression, ameliorating or palliating the disease state, and remission or improved prognosis. An individual is successfully "treated", for example, if one or more symptoms associated with a disorder (e.g., an eosinophil-mediated disease) are mitigated or eliminated. For example, an individual is successfully "treated" if treatment results in increasing the quality of life of those suffering from a disease, decreasing the dose of other medications required for treating the disease, reducing the frequency of recurrence of the disease, lessening severity of the disease, delaying the development or progression of the disease, and/or prolonging survival of individuals.

An "effective amount" refers to at least an amount effective, at dosages and for periods of time necessary, to achieve the desired or indicated effect, including a therapeutic or prophylactic result. An effective amount can be provided in one or more administrations. A "therapeutically effective amount" is at least the minimum dose of cells required to effect a measurable improvement of a particular disorder. In some embodiments, a therapeutically effective amount is the amount of a composition that reduces the severity, the duration and/or the symptoms associated with cancer, viral infection, microbial infection, or septic shock in an animal. A therapeutically effective amount herein may vary according to factors such as the disease state, age, sex, and weight of the patient. A therapeutically effective amount may also be one in which any toxic or detrimental effects of the antibody are outweighed by the therapeutically beneficial effects. A "prophylactically effective amount" refers to an amount effective, at the dosages and for periods of time necessary, to achieve the desired prophylactic result. Typically but not necessarily, since a prophylactic dose is used in subjects prior to or at the earlier stage of disease, the prophylactically effective amount can be less than the therapeutically effective amount.

As used herein, an "individual" or a "subject" is a mammal. A "mammal" for purposes of treatment includes humans, domestic and farm animals, and zoo, sports, or pet animals, such as dogs, horses, rabbits, cattle, pigs, hamsters, gerbils, mice, ferrets, rats, cats, *etc.* In some embodiments, the individual or subject is human.

### II. METHODS FOR OBTAINING AND PREPARING COMPOSITIONS CONTAINING NATURAL KILLER SUBSETS

Provided are methods for preparing compositions containing or enriched for primary CD16 158V+ NK cells or CD16 158V+/g- NK cells. Also provided are resulting compositions that contain or are enriched for primary CD16 158V+ or CD16 158V+/g- NK cell subsets. In some embodiments, the provided primary cells are enriched from a subject, such as a mammalian subject, for example a human subject. In some embodiments, provide are compositions containing or enriched for primary human CD16 158V+ NK cells or CD16 158V+/g- NK cells.

In some embodiments, the subject is a healthy subject. In some embodiments, the subject is a subject that has a disease of conditions, e.g. a cancer. In some embodiments, the compositions containing or enriched for primary CD16 158V+ NK cells or CD16 158V+/g- NK cells can be administered for allogenic or autologous adoptive cell therapy.

In some embodiments, methods for enriching NK cells includes (a) isolating NK cells or a subset thereof from a subject identified as having the CD16 158V+ NK cell genotype; and (b) culturing the isolated NK cells or subset thereof under conditions to expand the cells, thereby enriching NK cells or the subset thereof from the subject. In some embodiments, prior to step (a), the method includes screening subjects for the presence of the CD16 158V+ NK cell genotype.

In some embodiments, subjects are screened for CD16 158V+, i.e. screened for the presence of the V158F polymorphism in CD16 (SNP rs396991). In some embodiments, genomic DNA is extracted from a biological sample from the subject, such as blood sample or bone marrow sample. In some embodiments, the sample is or comprises blood cells, e.g. peripheral blood mononuclear cells. In some embodiments, the sample is a sample from a healthy donor subject. Any method for extracting DNA from the sample can be employed. For instance, nucleic acids can be readily isolated from a sample, e.g. cells, using standard techniques such as guanidium thiocyanate-phenol-chloroform extraction (Chomocyznski et al. (1987) Anal. Biochem. 162: 156). Commercially available kits also are readily available for extracting genomic DNA, such as the Wizard genomic DNA purification kit (Promega, Madison, WI).

Genotyping can be performed on any suitable sample. In any of the embodiments described herein, the genotyping reaction can be, for example, a pyrosequencing reaction, DNA sequencing reaction, MassARRAY MALDI- TOF, RFLP, allele-specific PCR, real-time allelic discrimination, or microarray. In some embodiments, a PCR-based technique, such as RT-PCR, of genomic DNA is carried out using allele-specific primers for the polymorphism. The PCR method for amplifying target nucleic acid sequences in a sample is well known in the art and has been described in, e.g., Innis et al. (eds.) PCR Protocols (Academic Press, NY 1990); Taylor (1991) Polymerase chain reaction: basic principles and automation, in PCR: A Practical Approach, McPherson et al. (eds.) IRL Press, Oxford; Saiki et al. (1986) Nature 324: 163; as well as in U.S. Patent Nos. 4,683,195, 4,683,202 and 4,889,818, all incorporated herein by reference in their entireties.

Primers for detecting the 158V+ polymorphism are known or can be easily designed by a skilled artisan, See. e.g. International published PCT Appl. No. WO2012/061814; Kim et al. (2006) Blood, 108:2720-2725; Cartron et al. (2002) Blood, 99:754-758; Koene et al. (1997) Blood, 90:1109-1114). In some embodiments, PCR can be carried out using nested primers followed by allele-specific restriction enzyme digestion. In some embodiments, the first PCR primers comprise nucleic acid sequences 5' -ATA TTT ACA GAA TGG CAC AGG -3' (SEQ ID NO:1) and 5'-GAC TTG GTA CCC AGG TTG AA-3' (SEQ ID NO:2), while the second PCR primers are 5'-ATC AGA TTC GAT CCT ACT TCT GCA GGG GGC AT-3' (SEQ ID NO:3) and 5'-ACG TGC TGA GCT TGA GTG ATG GTG ATG TTC AC-3' (SEQ ID NO:4), which, in some cases, generates a 94-bp fragment depending on the nature of allele. In some embodiments, the primer pair comprises the nucleic acid sequences set forth in SEQ ID NO:5 (CCCAACTCAA CTTCCCAGTG TGAT) and SEQ ID NO:6 (GAAATCTACC TTTTCCTCTA ATAGGGCAAT). In some embodiments, the primer pair comprises the nucleic acid sequences set forth in SEQ ID NO:5 (CCCAACTCAA CTTCCCAGTG TGAT) and SEQ ID NO:7 (GAAATCTACC TTTTCCTCTA ATAGGGCAA). In some embodiments, the primer pair comprises the nucleic acid sequences set forth in SEQ ID NO:5 (CCCAACTCAA CTTCCCAGTG TGAT) and SEQ ID NO:8 (GAAATCTACC TTTTCCTCTA ATAGGGCA).

The genomic sequence for CD16a is available in the NCBI database at NG_009066.1. The gene ID for CD16A is 2214. Sequence information for CD16, including gene polymorphisms, is available at UniProt Acc. No. P08637. The sequence of CD16 (F158) is set forth in SEQ ID NO:9 (residue F158 is bold and underlined). In some embodiments, CD16 (F158) further comprises a signal peptide set forth as MWQLLLPTALLLLVSA (SEQ ID NO:10).

GMRTEDLPKAVVFLEPQWYRVLEKDSVTLKCQGAYSPEDNSTQWFHNESLISSQASSYFIDA
ATVDDSGEYRCQTNLSTLSDPVQLEVHIGWLLLQAPRWVFKEEDPIHLRCHSWKNTALHKV
TYLQNGKGRKYFHHNSDFYIPKATLKDSGSYFCRGLFGSKNVSSETVNITITQGLAVSTISSFF
PPGYQVSFCLVMVLLFAVDTGLYFSVKTNIRSSTRDWKDHKFKWRKDPQDK (SEQ ID
NO:9)

The sequence of CD16 158V+ (polymorphism resulting in F158V) is known as VAR_003960 and has the sequence set forth in SEQ ID NO:11 (158V+ polymorphism is in bold and underline). In some embodiments, CD16 (158V+) further comprises a signal peptide set forth as MWQLLLPT ALLLL VSA (SEQ ID NO:10).

GMRTEDLPKAVVFLEPQWYRVLEKDSVTLKCQGAYSPEDNSTQWFHNESLISSQASSYFI
DAATVDDSGEYRCQTNLSTLSDPVQLEVHIGWLLLQAPRWVFKEEDPIHLRCHSWKNTA
LHKVTYLQNGKGRKYFHHNSDFYIPKATLKDSGSYFCRGLVGSKNVSSETVNITITQGLA
VSTISSFFPPGYQVSFCLVMVLLFAVDTGLYFSVKTNIRSSTRDWKDHKFKWRKDPQDK
(SEQ ID NO:11)

In some embodiments, the provided cells include or are enriched for CD16 158V+ NK cells that has the sequence of amino acids set forth in SEQ ID NO:11 or a sequence of amino acids that exhibits at least 85%, 86%, 87%, 88%, 89%, 90%, 91%,92%,93%, 94%, 95%, 96%, 97%, 98%, 99% or more sequence identity to SEQ ID NO:11 and that contains the 158V+ polymorphism. In some embodiments, the CD16 158V+ NK cells exhibit increased binding affinity for Fc, such as IgG Fc region, and/or is associated with increased tumor cell killing compared to NK cells expressing CD16 (F158) set forth in SEQ ID NO:9. In some embodiments, the increase is greater than 1.5-fold, 2-fold, 3-fold, 4-fold, 5-fold, 6-fold, 7-fold, 8-fold, 9-fold, 10-fold, 20-fold, 30-fold, 40-fold, 50-fold or more.

In some embodiments, single nucleotide polymorphism (SNP) analysis is employed on genomic deoxyribonucleic acid (DNA) samples using allele-specific probes containing a fluorescent dye label (e.g. FAM or VIC) on the 5' end and a minor groove binder (MGB) and nonfluorescent quencher (NFQ) on the 3' end and an unlabeled PCR primers to detect a specific SNP targets. In some embodiments, the assay measures or detects the presence of an SNP by a change in fluorescence of the dyes associated with the probe. In such embodiments, probes hybridize to the target DNA between the two unlabeled primers and signal from the fluorescent dye on the 5' end is quenched by the NFQ on its 3' end by fluorescence resonance energy transfer (FRET). During PCR, Taq polymerase extends the unlabeled primers using the template as a guide and when the polymerase reaches the labeled probe, it cleaves the molecule separating the dye from the quencher. In some aspects, a qPCR instrument can detect fluorescence from the unquenched label. Exemplary of such are commercially available SNP Assays, e.g. code C_25815666_10 for rs396991 (Applied Biosystems, Cat No. 4351379 for SNP genotyping of V158F in CD16).

In some embodiments, subjects heterozygous or homozygous for the CD16 158V+ (V158F) polymorphism are identified. In some embodiments, subjects homozygous for the CD16 158V+ (V158F) polymorphism are identified. In some embodiments, NK cells or an NK cell subset are isolated from subjects identified as being heterozygous or homozygous for the CD16 158 V+ polymorphism. In some embodiments, NK cells or an NK cell subset are isolated from subjects identified as being homozygous for the CD16 158 V+ polymorphism. Methods for isolating NK cells are well-known to a skilled artisan.

In some embodiments, natural killer cells are first isolated and then sorted using available procedures. For example, a population of lymphocytes or peripheral blood mononuclear cells (PBMCs) can be obtained. Natural killer cells expressing one or more natural killer cell-specific markers, such as described above, are isolated from the cell population. It is within the level of a skilled artisan to choose particular markers or combinations of surface markers. In some embodiments, the surface marker(s) is any one or more of the from the following surface antigens CD11a, CD3, CD7, CD14, CD16, CD19, CD25, CD27, CD56, CD57, CD161, CD226, NKB1, CD62L; CD244, NKG2D, NKp30, NKp44, NKp46, NKG2A, NKG2C, KIR2DL1 and/or KIR2DL3. Reagents, including fluorochrome-conjugated antibodies, for detecting such surface antigens are well known and available to a skilled artisan.

In some embodiments, non-NK cells are excluded based on positive surface expression of CD19 and/or CD14.

In some embodiments, NK cells are isolated as those present in a subpopulation gated as the CD3⁻CD56^{dim} population. In some embodiments, isolated NK cells are present in the subpopulation gated as CD3⁻CD56^{dim}CD14⁻CD19⁻.

In some embodiments, a g-NK cell subset is isolated or enriched from the subject. In some embodiments, g- NK cells are isolated or enriched from subjects identified as bearing the CD16 158V+ polymorphism, e.g. subjects identified as being heterozygous or homozygous for the polymorphism. In some embodiments, provided is a method of identifying g-NK cells from a subject bearing the CD16 158V+ polymorphism, e.g. subjects identified as being heterozygous or homozygous for the polymorphism, comprising: identifying a subset of natural killer cells in a population of lymphocytes from the subject that do not express substantial FcRy to thereby identify g-NK cells. In one embodiment, lymphocytes are obtained or isolated from the subject. In some embodiments, cells that do not express substantial FcRy but do express at least one marker for natural killer cells are identified.

An amino acid sequence for FcRy chain (*Homo sapiens,* also called the High affinity immunoglobulin gamma Fc receptor I) is available in the NCBI database as accession number NP_004097.1 (GI:4758344), and is reproduced below as SEQ ID NO:12.

MIPAVVLLLLLLVEQAAALGEPQLCYILDAILFLYGIVLT LLYCRLKIQVRKAAITSYEK
SDGVYTGLSTRNQETYETLKHEKPPQ (SEQ ID NO:12)

In some embodiments, isolation or enrichment of g-NK cells is carried out by immunoaffinity-based methods. In some embodiments, positive and/or negative strategies can be employed to enrich cells that are surface positive for one or more markers and/or that are surface negative or low for one or more markers, respectively. In some embodiments, isolation or enrichment is carried out by fluorescence-activated cell sorting (FACs) by collecting cells gated or positive for surface expression of one or more particular marker or markers (positive enrichment) and/or by excluding cells positive for surface expression of one or more particular marker or markers (negative enrichment). In some embodiments, isolation or enrichment is carried out by contacting the lymphocytes with a solid surface to which an antibody or antibodies specific for a marker is bound and either recovering cells not bound to the antibody (negative enrichment) or recovering cells bound to the antibody (positive enrichment).

In some aspects, g-NK cells are identified by the presence, absence or level of surface expression of one or more various marker that distinguishes NK cells from other lymphocytes or immune cells and that distinguishes g-NK cells from conventional NK cells. In some embodiments, g-NK cells can be isolated as described in published Patent Appl. No. US2013/0295044 or Zhang et al. (2013) J. Immunol., 190:1402-1406.

In some embodiments, a cell (e.g. NK cell subset) is positive for a particular marker if there is detectable presence on or in the cell of a particular marker, typically a surface marker. In embodiments, surface expression can be determined by flow cytometry, for example, by staining with an antibody that specifically bind to the marker and detecting the binding of the antibody to the marker, wherein surface expression is positive if staining is detectable at a level substantially above the staining detected carrying out the same procedures with an isotype-matched control under otherwise identical conditions and/or at a level substantially similar to, or in some cases higher than, a cell known to be positive for the marker and/or at a level higher than that for a cell known to be negative for the marker.

In some embodiments, a cell (e.g. NK cell subset) is negative for a particular marker if there is an absence of detectable presence on or in the cell of a particular marker, typically a surface marker. In embodiments, surface expression can be determined by flow cytometry, for example, by staining with an antibody that specifically bind to the marker and detecting the binding of the antibody to the marker, wherein surface expression is negative if staining is not detectable at a level substantially above the staining detected carrying out the same procedures with an isotype-matched control under otherwise identical conditions and/or at a level substantially lower than a cell known to be positive for the marker and/or at a level substantially similar to a cell known to be negative for the marker.

In some embodiments, a cell (e.g. NK cell subset) is low for a particular marker if there is a lower level of detectable presence on or in the cell of a particular marker, typically a surface marker, compared to a cell known to be positive for the marker. In embodiments, surface expression can be determined by flow cytometry, for example, by staining with an antibody that specifically bind to the marker and detecting the binding of the antibody to the marker, wherein surface expression is low if staining is at a level lower than a cell known to be positive for the marker.

In some embodiments, an NK cell known to be positive for a surface marker can be a conventional NK cell and/or a subset of NK cells that represent the majority of NK cells present in a subject or that represent the majority of NK cells present on average in a population of subjects.

In some embodiments, the g-NK cell subset of NK cells can be detected by observing whether FcRy is expressed by a population of NK cells or a subpopulation of NK cells. The NK cells that do not express FcRy are g-NK cells. In some embodiments, it may be useful to detect expression of any of the above mentioned other NK cell markers as a positive identifier that the cell is a natural killer cell while also confirming that the cell does not express FcRy. Expression of a natural killer cell surface marker that correlates with the expression of FcRy (or lack thereof) can be detected by any available procedure that does not injure the cells. For example, the g-NK cells can be detected, identified and/or isolated by flow cytometry by use of such a cell surface marker that correlates with FcRy expression.

The CD3ζ and/or FcRy proteins are intracellular proteins that are not easily detected unless the cells are treated to allow intracellular proteins to be detected, for example, by fixation and permeabilization. While such treatment can be used to confirm the identity of a substantially pure population of cells, in many cases cell-surface markers can be employed that can be detected without injuring the cells when identifying and isolating g-NK cells. In some embodiments, g-NK cells can be identified based on positive surface expression of a KIR (Killer cell immunoglobulin-like receptors) and/or based on low or negative surface expression of Natural cytotoxicity receptors (NCR).

In some embodiments, g-NK cells are positive for surface expression of a KIR (e.g. KIR2DL1, KIR2DL2/3 and/or KIR3DL1) and can be used to identify and isolate g-NK cells, such as by positive selection methods. In some embodiments, g-NK cells are surface positive for one or more of KIR2DL1, KIR2DL2/3 and KIR3DL1.

In some embodiments, NCR (e.g. Nkp30 and/or Nkp46), which are normally expressed on NK cells, are not expressed or are expressed at low levels on g-NK cells and can be used to identify and isolate g-NK cells, such as by negative selection methods. In some embodiments, g-NK cells are surface negative or low for NkP30 and/or Nkp46. In some embodiments, g-NK cells are surface positive for NKG2C. In some embodiments, g-NK cells express high CD57 and low NKG2A.

Antibodies and other binding entities can be used to detect expression levels of marker proteins (e.g., KIR and/or NCR), and isolate the g-NK cells. For example, in some embodiments, antibodies specific for FcRy-expressing cells (e.g., antibodies specific for a cell surface marker such as NCR that correlates with FcRy expression) can be bound to a solid substrate, natural killer cells can be contacted with the antibody-substrate, and cells that do not bind to the antibodies are collected. Other methods available to those of skill in the art can also be employed. Suitable antibodies may include polyclonal, monoclonal, fragments (such as Fab fragments), single chain antibodies and other forms of specific binding molecules.

In some embodiments, the natural killer cells may be identified based on surface expression of typical human natural killer cell markers such as KIR, NKG2A, NKG2D, NKp30, NKp44, NKp46, CD16, CD56, and CD161. In some embodiments, a subset of natural killer cells in a population of lymphocytes is identified comprising contacting a sample of lymphocytes with an antibody that binds to a natural killer cell-specific antigen. In one embodiment, the marker for natural killer cells is CD16, KIR2DL1, KIR2DL2/3, KIR3DL1, NKp30, NKp46 or a combination thereof. NK cells, including g- NK cells, can be isolated based on positive or negative/low surface expression of any of the above markers. In one method, the method involves isolating a substantially pure population of natural killer cells, such as based on surface expression of KIR, NKG2A, NKG2D, NKp30, NKp44, NKp46, CD16, CD56, and/or CD161.

In some embodiments, g-NK cells are identified as those that do not express or are low for surface expression of NCR protein. In some embodiments, the NCR is Nkp30 and/or Nkp46. For example, the population of cells containing natural killer cells can be sorted by flow cytometry using an antibody specific for NCR protein (e.g. Nkp30 and/or Nkp46). The cells that are labeled by the anti-NCR antibodies are discarded and the cells that are not labeled by the anti-NCR antibodies are retained as cells that are or are enriched for cells containing g-NK cells. Alternatively, the population of cells containing natural killer cells can be contacted with a solid substrate to which anti-NCR antibodies are bound. The cells that do not bind are or are enriched for g-NK cells. In another embodiment, the population of cells containing natural killer cells can be contacted with magnetic beads to which anti-NCR antibodies are bound, after incubation the magnetic beads are removed from the cell population, leaving cells that are or are enriched for g-NK cells as a substantially pure population of cells.

In some embodiments, g-NK cells are identified as those that express one or more KIR. In some embodiments, the KIR is KIR2DL1, KIR2DL2/3 and/or KIR3DL1. For example, the population of cells containing natural killer cells can be sorted by flow cytometry using an antibody specific for KIR protein (e.g. KIR2DL1, KIR2DL2/3 and/or KIR3DL1). The cells that are labeled by the anti-KIR antibodies are retained as a subset of NK cells that are or are enriched for g-NK cells and the cells that are not labeled by the anti-KIR antibodies are discarded. Alternatively, the population of cells containing natural killer cells can be contacted with a solid substrate to which anti-KIR antibodies are bound. The cells that bind are or are enriched for g-NK cells. In another embodiment, the population of cells containing natural killer cells can be contacted with magnetic beads to which anti-KIR antibodies are bound, after incubation the magnetic beads are recovered, thereby isolating or obtaining from the cell population cells that are or that are enriched for g-NK cells.

In some embodiments, g-NK cells are identified that are surface positive for CD16 and a KIR (e.g. KIR2DL1, KIR2DL2/3 and/or KIR3DL1) and/or that are surface negative or low for an NCR (e.g. NKp30 and/or NKp46), e.g. CD16⁺, KIR⁺, and NCR^{-/lo} (e.g. Nkp30^{-/lo} and/or Nkp46^{-/lo}). In some embodiments, the g-NK cells also exhibit a surface phenotype that is CD3⁻CD56^{dim}CD14⁻CD19⁻.

In some embodiments, following isolation or enrichment, cells enriched for CD16 158V+ NK cells or CD16 158V+/g-NK cells are cultured in a cell culture medium, such as for expanding the isolated or enriched cells. In some cases, it may be necessary or desirable to culture the NK cells to expand them prior to formulating them as a composition for administration. In some of the embodiments, methods of producing a composition comprising engineered NK cells comprises culturing or incubating the NK cells isolated or enriched for CD16 158V+ NK cells or CD16 158V+/g-NK cells, such as to expand the cells to a therapeutically effective amount prior to administering the NK cells to an individual in need thereof.

Suitable methods for culturing and expanding NK cells are known. For example, the NK cells may be cultured using feeder cells, or in the presence of cytokines to enhance their growth and/or activation. As used herein "culturing" includes providing the chemical and physical conditions (e.g., temperature, gas) which are required for NK cell maintenance, and growth factors. In one embodiment, culturing the NK cells includes providing the NK cells with conditions for proliferation. Examples of chemical conditions which may support NK cell proliferation include but are not limited to buffers, nutrients, serum, vitamins and antibiotics as well as cytokines and other growth factors which are typically provided in the growth (i.e., culture) medium. In one embodiment, the NK culture medium includes MEMα comprising 10% FCS or CellGro SCGM (Cell Genix) comprising 5% Human Serum/LiforCell^{®} FBS Replacement (Lifeblood Products). Other media suitable for use with the invention include, but are not limited to Glascow's medium (Gibco Carlsbad Calif.), RPMI medium (Sigma-Aldrich, St Louis Mo.) or DMEM (Sigma-Aldrich, St Louis Mo.). It will be noted that many of the culture media contain nicotinamide as a vitamin supplement for example, MEMα (8.19 µM nicotinamide), RPMI (8.19 µM nicotinamide), DMEM (32.78 µM nicotinamide) and Glascow's medium (16.39 µM nicotinamide).

In some embodiments, such as for applications in which cells are introduced (or reintroduced) into a human subject, culturing is carried out using serum-free formulations, such as AIM V^{™} serum free medium for lymphocyte culture or MARROWMAX^{™} bone marrow medium. Such medium formulations and supplements are available from commercial sources such as Invitrogen (GIBCO) (Carlsbad, Calif.). The cultures can be supplemented with amino acids, antibiotics, and/or with cytokines to promote optimal viability, proliferation, functionality and/or and survival.

In some embodiments, culturing the population of cells comprising the engineered NK cells is effected without a feeder layer or feeder cells. In some of these embodiments, the engineered NK cells can be cultured with a growth factor. According to some embodiments, the at least one growth factor comprises a growth factor selected from the group consisting of SCF, FLT3, IL-2, IL-7, IL-15, IL-12 and IL-21. According to some embodiments, the at least one growth factor is IL-2 or IL-7 and IL-15. According to some embodiments, the at least one growth factor is solely IL-2. In some embodiments, cells are cultured for 3 days to 30 days, such as for 7 to 28 days, for example for at least or at least about 7, 8, 9, 10, 11, 12, 13, 14, 15, 16, 17, 18 or more days.

In some embodiments, IL-12, IL-15, IL-18, IL-2, and/or CCL5 is administered to a subject prior to isolating or enriching for CD16 158V+ NK cells or CD16 158V+/g-NK cells.

In some embodiments, the cells can be isolated from a subject chosen to express an HLA allele of a desired MHC (HLA) restriction. In some embodiments, HLA typing of cells prior to or after isolation or enrichment can be carried out. In some embodiments, the HLA typing of cells, including primary cells obtained from a subject, can be determined using procedures well known in the art, such as by performing tissue typing using molecular haplotype assays (BioTest ABC SSPtray, BioTest Diagnostics Corp., Denville, NJ; SeCore Kits, Life Technologies, Grand Island, NY). In some cases, it is well within the level of a skilled artisan to perform standard typing of cells to determine the HLA genotype, such as by using sequence-based typing (SBT) (Adams et al., (2004) J. Transl. Med., 2:30; Smith (2012) Methods Mol Biol., 882:67-86).

### III. COMPOSITIONS AND KITS

Provided herein are compositions comprising the cells enriched for CD16 158V+ NK cells or CD16 158V+/g- NK cells. In some embodiments, the composition comprises about 5-99% CD16 158V+ NK cells or CD16 158V+/g- NK cells, or any percentage of CD16 158V+ NK cells or CD16 158V+/g- NK cells between 5 and 99% inclusive. In some embodiments, the composition can include an increased or greater percentages of CD16 158V+ NK cells or CD16 158V+/g- NK cells relative to total NK cells or total cells compared to the percentage of CD16 158V+ NK cells or CD16 158V+/g- NK relative to total NK cells or total cells naturally present in the subject from which the cells were isolated. In some embodiments, the percentage is increased at least or at least about 2-fold, 3-fold, 4-fold, 5-fold, 10-fold, 20-fold, 30-fold, or more.

In some embodiments, the composition can include 20%, 30%, 40%, 50%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99%, or substantially 100% CD16 158V+ NK cells or CD16 158V+/g- NK cells. In some embodiments, the composition comprises more than 50% CD16 158V+ NK cells or CD16 158V+/g- NK cells. In another embodiment, the composition comprises more than 70% CD16 158V+ NK cells or CD16 158V+/g- NK cells. In another embodiment, the composition comprises more than 80% CD16 158V+ NK cells or CD16 158V+/g- NK cells. In some embodiments, the provided compositions include those in which the CD16 158V+ NK cells or CD16 158V+/g- NK cells make up at least or at least about 60%, 70%, 80%, 85%, 90%, 95% or more of the cells in the composition or of the NK cells in the composition.

Among the compositions are pharmaceutical compositions and formulations for administration, such as for adoptive cell therapy. In some embodiments, the engineered cells are formulated with a pharmaceutically acceptable carrier.

A pharmaceutically acceptable carrier can include all solvents, dispersion media, coatings, antibacterial and antifungal agents, isotonic and absorption delaying agents, and the like, compatible with pharmaceutical administration (Gennaro, 2000, Remington: The science and practice of pharmacy, Lippincott, Williams & Wilkins, Philadelphia, PA). Examples of such carriers or diluents include, but are not limited to, water, saline, Ringer's solutions, dextrose solution, and 5% human serum albumin. Liposomes and non-aqueous vehicles such as fixed oils may also be used. Supplementary active compounds can also be incorporated into the compositions. The pharmaceutical carrier should be one that is suitable for NK cells, such as a saline solution, a dextrose solution or a solution comprising human serum albumin.

In some embodiments, the pharmaceutically acceptable carrier or vehicle for such CD16 158V+ NK cells or CD16 158V+/g- NK cells is any non-toxic aqueous solution in which the CD16 158V+ NK cells or CD16 158V+/g- NK cells can be maintained, or remain viable, for a time sufficient to allow administration of live CD16 158V+ NK cells or CD16 158V+/g- NK cells. For example, the pharmaceutically acceptable carrier or vehicle can be a saline solution or buffered saline solution. The pharmaceutically acceptable carrier or vehicle can also include various bio materials that may increase the efficiency of CD16 158V+ NK cells or CD16 158V+/g- NK cells. Cell vehicles and carriers can, for example, include polysaccharides such as methylcellulose (M. C. Tate, D. A. Shear, S. W. Hoffman, D. G. Stein, M. C.

LaPlaca, Biomaterials 22, 1113, 2001, which is incorporated herein by reference in its entirety), chitosan (Suh J K F, Matthew H W T. Biomaterials, 21, 2589, 2000; Lahiji A, Sohrabi A, Hungerford D S, et al., J Biomed Mater Res, 51, 586, 2000, each of which is incorporated herein by reference in its entirety), N-isopropylacrylamide copolymer P(NIPAM-co-AA) (Y. H. Bae, B. Vernon, C. K. Han, S. W. Kim, J. Control. Release 53, 249, 1998; H. Gappa, M. Baudys, J. J. Koh, S. W. Kim, Y. H. Bae, Tissue Eng. 7, 35, 2001, each of which is incorporated herein by reference in its entirety), as well as Poly(oxyethylene)/poly(D,L-lactic acid-co-glycolic acid) (B. Jeong, K. M. Lee, A. Gutowska, Y. H. An, Biomacromolecules 3, 865, 2002, which is incorporated herein by reference in its entirety), P(PF-co-EG) (Suggs L J, Mikos A G. Cell Trans, 8, 345, 1999, which is incorporated herein by reference in its entirety), PEO/PEG (Mann B K, Gobin A S, Tsai A T, Schmedlen R H, West J L., Biomaterials, 22, 3045, 2001; Bryant S J, Anseth K S. Biomaterials, 22, 619, 2001, each of which is incorporated herein by reference in its entirety), PVA (Chih-Ta Lee, Po-Han Kung and Yu-Der Lee, Carbohydrate Polymers, 61, 348, 2005, which is incorporated herein by reference in its entirety), collagen (Lee C R, Grodzinsky A J, Spector M., Biomaterials 22, 3145, 2001, which is incorporated herein by reference in its entirety), alginate (Bouhadir K H, Lee K Y, Alsberg E, Damm K L, Anderson K W, Mooney D J. Biotech Prog 17, 945, 2001; Smidsrd O, Skjak-Braek G., Trends Biotech, 8, 71, 1990, each of which is incorporated herein by reference in its entirety).

In some embodiments, the CD16 158V+ NK cells or CD16 158V+/g- NK cells can be present in the composition in an effective amount. An effective amount of activated CD16 158V+ NK cells or CD16 158V+/g- NK cells can vary depending on the patient, as well as the type, severity and extent of disease. Thus, a physician can determine what an effective amount is after considering the health of the subject, the extent and severity of disease, and other variables.

In certain embodiments, the number of cells in the composition provides the CD16 158V+ NK cells or CD16 158V+/g- NK cells at a therapeutically effective amount. In some embodiments, the amount is an amount that reduces the severity, the duration and/or the symptoms associated with cancer, viral infection, microbial infection, or septic shock in an animal. In certain other embodiments, a therapeutically effective amount is a dose of cells that results in a reduction of the growth or spread of cancer by at least 2.5%, at least 5%, at least 10%, at least 15%, at least 25%, at least 35%, at least 45%, at least 50%, at least 75%, at least 85%, by at least 90%, at least 95%, or at least 99% in a patient or an animal administered a composition described herein relative to the growth or spread of cancer in a patient (or an animal) or a group of patients (or animals) not administered the composition. In some embodiments, an effective amount for cytotoxicity is defined as an amount of NK cells that is able to inhibit or reduce the growth of cancer, viral and microbial cells.

In some embodiments, the composition comprises a dose of a composition enriched for or containing CD16 158V+ NK cells or CD16 158V+/g- NK cells that is from or from about 10⁵ to about 10¹² cells, or about 10⁵ to about 10⁸ cells, or about 10⁶ to about 10¹² cells, or about 10⁸ to about 10¹¹ cells, or about 10⁹ to about 10¹⁰ cells. In some embodiments, the composition comprises greater than or greater than about 10⁵, about 10⁶, about 10⁷, about 10⁸, about 10⁹, about10¹⁰, about 10¹¹, or about 10¹² cells. In some embodiments, such an amount can be administered to a cancer patient.

In some embodiments, the volume of the composition is at least or at least about 10 mL, 50 mL, 100 mL, 200 mL, 300 mL, 400 mL or 500 mL, such as is from or from about 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL, each inclusive. In some embodiments, the composition has a cell density of at least or at least about 1 x 105 cells/mL, 5 × 10⁵ cells/mL, 1 × 10⁶ cells/mL, 5 × 10⁶ cells/mL, 1 × 10⁷ cells/mL, 5 × 10⁷ cells/mL or 1 × 10⁸ cells/ mL. In some embodiment, the cell density of the composition is between or between about 1 × 10⁵ cells/mL to 1 × 10⁸ cells/mL, 1 × 10⁵ cells/mL to 1 × 10⁷ cells/mL, 1 × 10⁵ cells/mL to 1 × 10⁶ cells/mL, 1 × 10⁶ cells/mL to 1 × 10⁷ cells/mL, 1 × 10⁶ cells/mL to 1 × 10⁸ cells/mL, 1 × 10⁶ cells/mL to 1 × 10⁷ cells/mL or 1 × 10⁷ cells/mL to 1 × 10⁸ cells/mL, each inclusive.

In some embodiments, the composition, including pharmaceutical composition, is sterile. In some embodiments, isolation or enrichment of the cells is carried out in a closed or sterile environment, for example, to minimize error, user handling and/or contamination. In some embodiments, sterility may be readily accomplished, e.g., by filtration through sterile filtration membranes.

Also provided herein are compositions that are suitable for cryopreserving the provided NK cells. In some embodiments, the composition comprises NK cells that are or are enriched for CD16 158V+ NK cells or CD16 158V+/g- NK cells and a cryoprotectant. In some embodiments, the cryoprotectant is or comprises DMSO and/or s glycerol. In some embodiments, compositions formulated for cryopreservation can be stored at low temperatures, such as ultra low temperatures, for example, storage with temperature ranges from -40 °C to - 150 °C, such as or about 80 °C ± 6.0 ° C.

In some embodiments, the compositions can be preserved at ultra low temperature before the administration to a patient. The compositions can also be preserved at ultra low temperature after isolation from a mammalian subject and prior to expansion and/or administration to a subject. For example, CD16 158V+ NK cells or CD16 158V+/g- NK cells can be isolated or enriched, stored at ultra low temperature and then processed or expanded to yield prior to administration to a subject. Alternatively, CD16 158V+ NK cells or CD16 158V+/g- NK cells can be isolated, processed or expanded and then stored at ultra-low temperature prior to administration to a subject.

A typical method for the preservation at ultra low temperature in small scale is described, for example, in U.S. Pat. No. 6,0168,991. For small-scale, cells can be preserved at ultra low temperature by low density suspension (e.g., at a concentration of about 200×106/ml) in 5% human albumin serum (HAS) which is previously cooled. An equivalent amount of 20% DMSO can be added into the HAS solution. Aliquots of the mixture can be placed into vials and frozen overnight inside an ultra low temperature chamber at about -80° C.

In some embodiments, the cryopreserved NK cells are prepared for administration by thawing. In some cases, the NK cells can be administered to a subject immediately after thawing. In such an embodiment, the composition is ready-to-use without any further processing. In other cases, the NK cells are further processed after thawing, such as by resuspension with a pharmaceutically acceptable carrier, incubation with an activating or stimulating agent, or are activated washed and resuspended in a pharmaceutically acceptable buffer prior to administration to a subject.

Kits comprising the provided compositions enriched for or containing CD16 158V+ NK cells or CD16 158V+/g- NK cells are also provided herein. For example, in some embodiment provided herein is a kit comprising CD16 158V+ NK cells or CD16 158V+/g- NK cells and an additional agent. In some embodiments, the additional agent comprises an Fc domain. In some embodiment the additional agent is an Fc fusion protein or an antibody. In some embodiments, the additional agent is a human, humanized, or chimeric antibody. In some of these embodiments, the additional agent is a full length antibody. Exemplary antibodies are described below.

### IV. METHODS OF TREATMENT

In some embodiments, provided herein is a method of treating a condition in an individual, comprising administering a composition comprising or enriched for CD16 158V+ NK cells or CD16 158V+/g- NK cells to an individual in need thereof, such as any of the compositions described.

In some embodiments, the method comprises administering an effective amount of a composition containing CD16 158V+ NK cells or CD16 158V+/g- NK cells to an individual. In some embodiments, from or from about 10⁵ to about 10¹² cells, or about 10⁵ to about 10⁸ cells, or about 10⁶ to about 10¹² cells, or about 10⁸ to about 10¹¹ cells, or about 10⁹ to about 10¹⁰ cells are administered to the subject. In some embodiments, about 10⁵, about 10⁶, about 10⁷, about 10⁸, about10⁹, about10¹⁰, about 10¹¹, or about 10¹² cells are administered to the individual. In some embodiments, the number of cells is with reference to the number of CD16 158V+ NK cells/kg or CD16 158V+/g- NK cells in the administered composition. In some embodiments, from or from about 10⁶ to 10¹⁰ CD16 158V+ NK cells/kg or CD16 158V+/g- NK cells /kg are administered to the subject.

In some embodiments, the CD16 158V+ NK cells or CD16 158V+/g- NK cells are administered to an individual soon after isolation. In some embodiments, the CD16 158V+ NK cells or CD16 158V+/g- NK cells are administered to an individual within 1, 2, 3, 4, 5, 6, 7, 14, 21, or 28 days of isolation and the engineering.

In other embodiments, the CD16 158V+ NK cells or CD16 158V+/g- NK cells are stored or expanded by growth in culture prior to administration, such as by methods described above. For example, the NK cells can be stored for greater than 6, 12, 18, or 24 months prior to administration to the individual.

In some embodiments, the provided CD16 158V+ NK cells or CD16 158V+/g- NK cells and compositions can be can be administered to a subject by any convenient route including parenteral routes such as subcutaneous, intramuscular, intravenous, and/or epidural routes of administration.

The provided CD16 158V+ NK cells or CD16 158V+/g- NK cells and compositions can be used in methods of treating an individual with a tumor or hyperproliferative disorders or microbial infection such as a viral infection, yeast infection, fungal infection, protozoan infection and/or bacterial infection. The disclosed methods of treating a subject with the provided CD16 158V+ NK cells or CD16 158V+/g- NK cells and compositions can be in combination with a therapeutic monoclonal antibody, such as an anti-tumor antigen or anti-cancer antibody, anti-viral antibody or anti-bacterial antibody. The provided CD16 158V+ NK cells or CD16 158V+/g- NK cells and compositions can be administered for treatment of animals, such as mammalian animals, for example human subjects.

In some examples, the methods include treating a hyperproliferative disorder, such as a hematological malignancy or a solid tumor. Examples of types of cancer and proliferative disorders that can be treated with the compositions described herein include, but are not limited to, leukemia (e.g., myeloblastic, promyelocytic, myelomonocytic, monocytic, erythroleukemia, chronic myelocytic (granulocytic) leukemia, and chronic lymphocytic leukemia), lymphoma (e.g., Hodgkin's disease and non-Hodgkin's disease), fibrosarcoma, myxosarcoma, liposarcoma, chondrosarcoma, osteogenic sarcoma, angiosarcoma, endotheliosarcoma, Ewing's tumor, colon carcinoma, pancreatic cancer, breast cancer, ovarian cancer, prostate cancer, squamous cell carcinoma, basal cell carcinoma, adenocarcinoma, renal cell carcinoma, hepatoma, Wilm's tumor, cervical cancer, uterine cancer, testicular tumor, lung carcinoma, small cell lung carcinoma, bladder carcinoma, epithelial carcinoma, glioma, astrocytoma, oligodendroglioma, melanoma, neuroblastoma, retinoblastoma, dysplasia and hyperplasia. The treatment and/or prevention of cancer includes, but is not limited to, alleviating one or more symptoms associated with cancer, the inhibition or reduction of the progression of cancer, the promotion of the regression of cancer, and/or the promotion of the immune response.

In some examples, the methods include treating a viral infection, such as an infection caused by the presence of a virus in the body. Viral infections include chronic or persistent viral infections, which are viral infections that are able to infect a host and reproduce within the cells of a host over a prolonged period of time-usually weeks, months or years, before proving fatal. Viruses giving rise to chronic infections that which may be treated in accordance with the present invention include, for example, the human papilloma viruses (HPV), Herpes simplex, and other herpes viruses, the viruses of hepatitis B and C as well as other hepatitis viruses, human immunodeficiency virus, and the measles virus, all of which can produce important clinical diseases. Prolonged infection may ultimately lead to the induction of disease which may be, e.g., in the case of hepatitis C virus liver cancer, fatal to the patient. Other chronic viral infections which may be treated in accordance with the present invention include Epstein Barr virus (EBV), as well as other viruses such as those which may be associated with tumors.

Examples of viral infections which can be treated or prevented with the compositions and methods described herein include, but are limited to, viral infections caused by retroviruses (e.g., human T-cell lymphotrophic virus (HTLV) types I and II and human immunodeficiency virus (HIV)), herpes viruses (e.g., herpes simplex virus (HSV) types I and II, Epstein-Ban virus and cytomegalovirus), arenaviruses (e.g., lassa fever virus), paramyxoviruses (e.g., morbillivirus virus, human respiratory syncytial virus, and pneumovirus), adenoviruses, bunyaviruses (e.g., hantavirus), cornaviruses, filoviruses (e.g., Ebola virus), flaviviruses (e.g., hepatitis C virus (HCV), yellow fever virus, and Japanese encephalitis virus), hepadnaviruses (e.g., hepatitis B viruses (HBV)), orthomyoviruses (e.g., Sendai virus and influenza viruses A, B and C), papovaviruses (e.g., papillomaviruses), picornaviruses (e.g., rhinoviruses, enteroviruses and hepatitis A viruses), poxviruses, reoviruses (e.g., rotaviruses), togaviruses (e.g., rubella virus), and rhabdoviruses (e.g., rabies virus). The treatment and/or prevention of a viral infection includes, but is not limited to, alleviating one or more symptoms associated with said infection, the inhibition, reduction or suppression of viral replication, and/or the enhancement of the immune response.

In some embodiments, the provided CD16 158V+ NK cells or CD16 158V+/g- NK cells and compositions are used in a method of treating a yeast or bacterial infection. For example, the provided CD16 158V+ NK cells or CD16 158V+/g- NK cells and compositions and methods described herein can treat infections relating to Streptococcus pyogenes, Streptococcus pneumoniae, Neisseria gonorrhoea, Neisseria meningitidis, Corynebacterium diphtheriae, Clostridium botulinum, Clostridium perfringens, Clostridium tetani, Haemophilus influenzae, Klebsiella pneumoniae, Klebsiella ozaenae, Klebsiella rhinoscleromotis, Staphylococcus aureus, Vibrio cholera, Escherichia coli, Pseudomonas aeruginosa, Campylobacter (Vibrio) fetus, Campylobacterjejuni, Aeromonas hydrophila, Bacillus cereus, Edwardsiella tarda, Yersinia enterocolitica, Yersinia pestis, Yersinia pseudotuberculosis, Shigella dysenteriae, Shigella flexneri, Shigella sonnei, Salmonella typhimurium, Treponema pallidum, Treponemapertenue, Treponema carateneum, Borrelia vincentii, Borrelia burgdorferi, Leptospira icterohemorrhagiae, Mycobacterium tuberculosis, Toxoplasma gondii, Pneumocystis carinii, Francisella tularensis, Brucella aborts, Brucella suis, Brucella melitensis, Mycoplasma spp., Rickettsiaprowazeki, Rickettsia tsutsugumushi, Chlamydia spp., Helicobacter pylori or combinations thereof.

### Combination Therapy

In some embodiments, the provided CD16 158V+ NK cells or CD16 158V+/g- NK cells exhibit enhanced activity when activated by or contacted with antibodies or Fc-containing proteins. For example, the CD16 158V+ NK cells or CD16 158V+/g- NK cells can be activated by antibody-mediated crosslinking of CD16 or by antibody-coated tumor cells.

In some embodiments, provided herein is a method of treating a condition in an individual comprising administering an CD16 158V+ NK cells or CD16 158V+/g- NK cells or composition thereof and an antibody to a subject. One of ordinary skill in the art can select an appropriate therapeutic (e.g., anti-cancer) monoclonal antibody to administer to the subject with the provided CD16 158V+ NK cells or CD16 158V+/g- NK cells and compositions described herein, such as depending on the particular disease or condition of the individual. Suitable antibodies may include polyclonal, monoclonal, fragments (such as Fab fragments), single chain antibodies and other forms of specific binding molecules.

In some embodiments, the antibody may further include humanized or human antibodies. Humanized forms of non-human antibodies are chimeric Igs, Ig chains or fragments (such as Fv, Fab, Fab', F(ab')2 or other antigen-binding subsequences of an antibody) that contain minimal sequence derived from non-human Ig. In some embodiments, the antibody comprises an Fc domain.

Generally, a humanized antibody has one or more amino acid residues introduced from a non-human source. These non-human amino acid residues are often referred to as "import" residues, which are typically taken from an "import" variable domain. Humanization is accomplished by substituting rodent CDRs or CDR sequences for the corresponding sequences of a human antibody (Jones et al., 1986; Riechmann et al., 1988; Verhoeyen et al., 1988). Such "humanized" antibodies are chimeric antibodies (1989), wherein substantially less than an intact human variable domain has been substituted by the corresponding sequence from a non-human species. In practice, humanized antibodies are typically human antibodies in which some CDR residues and possibly some Fc residues are substituted by residues from analogous sites in rodent antibodies. Humanized antibodies include human antibodies (recipient antibody) in which residues from a complementary determining region (CDR) of the recipient are replaced by residues from a CDR of a non-human species (donor antibody) such as mouse, rat or rabbit, having the desired specificity, affinity and capacity. In some instances, corresponding non-human residues replace Fv framework residues of the human antibody. Humanized antibodies may comprise residues that are found neither in the recipient antibody nor in the imported CDR or framework sequences. In general, the humanized antibody comprises substantially all of at least one, and typically two, variable domains, in which most if not all of the CDR regions correspond to those of a non-human Ig and most if not all of the FR regions are those of a human antibody consensus sequence. The humanized antibody optimally also comprises at least a portion of an antibody constant region (Fc), typically that of a human antibody (Jones et al., 1986; Presta, 1992; Riechmann et al., 1988).

Human antibodies can also be produced using various techniques, including phage display libraries (Hoogenboom et al., 1991; Marks et al., 1991) and the preparation of human mAbs (Boerner et al., 1991; Reisfeld and Sell, 1985). Similarly, introducing human Ig genes into transgenic animals in which the endogenous antibody genes have been partially or completely inactivated can be exploited to synthesize human Abs. Upon challenge, human antibody production is observed, which closely resembles that seen in humans in all respects, including gene rearrangement, assembly, and antibody repertoire (1997a; 1997b; 1997c; 1997d; 1997; 1997; Fishwild et al., 1996; 1997; 1997; 2001; 1996; 1997; 1997; 1997; Lonberg and Huszar, 1995; Lonberg et al., 1994; Marks et al., 1992; 1997; 1997; 1997).

Specifically, the cells of the present invention can be targeted to tumors by administration with an antibody that recognizes a tumor associated antigen. One of ordinary skill in the art will appreciate that the present CD16 158V+ NK cells or CD16 158V+/g- NK cells are suitable for use with a wide variety of antibodies that recognize tumor associated antigens. Non-limiting examples of a tumor associated antigen includes CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin. In some cases, the antibody is an anti-CD20 antibody, an anti-HER2 antibody, an anti-CD52 antibody, an anti-EGFR antibody and an anti-CD38 antibody. Exemplary antibodies include rituximab, trastuzumab, aletuzumab, certuximab, daratumumab, veltuzumab, ofatumumab, ublituximab, ocaratuzumab. Antibodies specific for a selected cancer type can be chosen, and include any antibody approved for treatment of cancer. Examples include trastuzumab (Herceptin) for breast cancer, rituximab (Rituxan) for lymphoma, and cetuximab (Erbitux) for head and neck squamous cell carcinoma. A skilled artisan is familiar with FDA-approved monoclonal antibodies able to bind particular tumor or disease antigens, any of which can be used in accord with the provided methods for treating the tumor or disease.

The CD16 158V+ NK cells or CD16 158V+/g- NK cells and the additional agent can be administered sequentially or simultaneously. In some embodiments, the additional agent can be administered before administration of the CD16 158V+ NK cells or CD16 158V+/g- NK cells. In some embodiments, the additional agent can be administered after administration of the CD16 158V+ NK cells or CD16 158V+/g- NK cells. For example, the CD16 158V+ NK cells or CD16 158V+/g- NK cells can be administered simultaneously with antibodies specific for a selected cancer type. Alternatively, the CD16 158V+ NK cells or CD16 158V+/g- NK cells can be administered at selected times that are distinct from the times when antibodies specific for a selected cancer type are administered.

In particular examples, the subject is administered an effective dose of an antibody before, after, or substantially simultaneously with the population of CD16 158V+ NK cells or CD16 158V+/g- NK cells. In some examples, the subject is administered about 0.1 mg/kg to about 100 mg/kg of the antibody (such as about 0.5- 10 mg/kg, about 1-20 mg/kg, about 10-50 mg/kg, about 20-100 mg/kg, for example, about 0.5 mg/kg, about 1 mg/kg, about 2 mg/kg, about 3 mg/kg, about 4 mg/kg, about 5 mg/kg, about 8 mg/kg, about 10 mg/kg, about 16 mg/kg, about 20 mg/kg, about 24 mg/kg, about 36 mg/kg, about 48 mg/kg, about 60 mg/kg, about 75 mg/kg, or about 100 mg/kg). An effective amount of the antibody can be selected by a skilled clinician, taking into consideration the particular antibody, the particular disease or conditions (e.g. tumor or other disorder), the general condition of the subject, any additional treatments the subject is receiving or has previously received, and other relevant factors. The subject is also administered a population of CD16 158V+ NK cells or CD16 158V+/g- NK cells described herein. Both the antibody and the population of CD16 158V+ NK cells or CD16 158V+/g- NK cells are typically administered parenterally, for example intravenously; however, injection or infusion to a tumor or close to a tumor (local administration) or administration to the peritoneal cavity can also be used. One of skill in the art can determine appropriate routes of administration.

The CD16 158V+ NK cells or CD16 158V+/g- NK cells can also be administered simultaneously or sequentially with anti-microbial, anti-viral and other therapeutic agents. In some embodiments provided herein, the CD16 158V+ NK cells or CD16 158V+/g- NK cells can be administered to an individual in combination with cytokines and/or growth factors. According to some embodiments, the at least one growth factor comprises a growth factor selected from the group consisting of SCF, FLT3, IL-2, IL-7, IL-15, IL-12 and IL-21. In some embodiments, the CD16 158V+ NK cells or CD16 158V+/g- NK cells and the cytokines or growth factors are administered sequentially. For example, the CD16 158V+ NK cells or CD16 158V+/g- NK cells may be administered first, followed by administration of the cytokines and/or growth factors. In some embodiments, the CD16 158V+ NK cells or CD16 158V+/g-NK cells are administered simultaneously with the cytokines or growth factors.

In some embodiments, the subject is administered one or more cytokines (such as IL-2, IL- 15, IL-21, and/or IL-12) to support survival and/or growth of NK cells. The cytokine(s) can be administered before, after, or substantially simultaneously with the NK cells. In some examples, the cytokine(s) can be administered after the NK cells. In one specific example, the cytokine(s) is administered to the subject within about 1-8 hours (such as within about 1-4 hours, about 2-6 hours, about 4-6 hours, or about 5-8 hours) of the administration of the NK cells.

In some embodiments, the provided methods also can include administering CD16 158V+ NK cells or CD16 158V+/g- NK cells to an individual in combination with a chemotherapeutic agent. In some embodiments, the chemotherapeutic agent may comprise cyclophosphamide, fludarabine, methyl prednasone In some embodiments, the chemotherapeutic agent is selected from the group consisting of: thalidomide, cisplatin (cis-DDP), oxaliplatin, carboplatin, anthracenediones, mitoxantrone; hydroxyurea, methylhydrazine derivatives, procarbazine (N-methylhydrazine, MM), adrenocortical suppressants, mitotane (.omicron.,.rho.'-DDD), aminoglutethimide, RXR agonists, bexarotene, tyrosine kinase inhibitors, imatinib, mechlorethamine, cyclophosphamide. ifosfamide, melphalan (L-sarcolysin), chlorambucil, ethylenimines, methylmelamines, hexamethylmelamine, thiotepa, busulfan, carmustine (BCNU), semustine (methyl-CCNTJ), lomustine (CCNU), streptozocin (streptozotocin), DNA synthesis antagonists, estramustine phosphate, triazines, dacarbazine (OTIC, dimethyl-triazenoimidazolecarboxamide), temozolomide, folic acid analogs, methotrexate (amethopterin), pyrimidine analogs, fiuorouracin (5-fluorouracil, 5-FU, 5FTJ), floxuridine (fluorodeox>'uridine, FUdR), cytarabine (cytosine arabinoside), gemcitabine, purine analogs, mercaptopurine (6-mercaptopurine, 6- MP), thioguanine (6-thioguanine, TG), pentostatin (2'-deoxycoformycin, deoxycoformycin), cladribine and fludarabine, topoisomerase inhibitors, amsacrine, vinca alkaloids, vinblastine (VLB), vincristine, taxanes, paclitaxel, protein bound paclitaxel (Abraxane(R)), docetaxel (Taxotere(R)); epipodophyllotoxins, etoposide, teniposide, camptothecins, topotecan, irinotecan, dactinomycin (actinomycin D), daunorubicin (daunomycin, rubidomycin), doxorubicin, bleomycin, mitomycin (mitomycin C), idarubicin, epirubicin, buserelin, adrenocorticosteroids, prednisone, progestins , hydroxyprogesterone caproate, medroxyprogesterone acetate, megestrol acetate, diethylstilbestrol, ethinyl estradiol, tamoxifen, anastrozole; testosterone propionate, fluoxymesterone, flutamide, bicalutamide, and leuprolide.

In some embodiments, the cancer drug is thalidomide or its derivatives. In some embodiments, the cancer drug is selected from the group consisting of cisplatin, carboplatin, and oxaliplatin. In certain embodiments, the cancer drug is selected from the group consisting of paclitaxel, Abraxane(R), and Taxotere(R). In one embodiment, the chemotherueptic agent is selected from the group consisting of asparaginase, bevacizumab, bleomycin, doxorubicin, epirubicin, etoposide, 5-fluorouracil, hydroxyurea, streptozocin, and 6- mercaptopurine, cyclophosphamide, paclitaxel, and gemcitabine.

### V. EXEMPLARY EMBODIMENTS

Among the provided embodiments are:
1. A composition comprising a natural killer (NK) cell subset surface positive for CD16 158V+, wherein at least about 50%, at least about 60%, at least about 70%, at least about 80%, or at least about 90% of the cells in the composition comprise the CD16 158V+ NK cell subset.
2. The composition of embodiment 1, wherein the CD16 158V+ NK cell subset does not express FcRy chain (CD16 158V+/g-), is surface positive for a KIR and/or is surface negative or low for Nkp30 and/or Nkp46.
3. A composition comprising a natural killer (NK) cell subset surface positive for CD16 158V+ and deficient in expression of FcRy chain (CD16 158V+/g-), wherein at least about 50%, at least about 60%, at least about 70%, at least about 80%, or at least about 90% of the cells in the composition comprise the CD16 158V+/g- NK cell subset.
4. The composition of any of embodiments 1-3, wherein CD16 158V+ comprises the sequence set forth in SEQ ID NO:11 or a sequence that exhibits at least 85%, 90% or 95% sequence identity to SEQ ID NO:11 and comprises the 158V+ polymorphism.
5. The composition of any of embodiments 1-4, wherein the NK cell subset is surface positive for a KIR, optionally KIR2DL2/3.
6. The composition of any of embodiments 1-5, wherein the NK cell subset is negative or low for surface expression of Nkp30 and/or Nkp46.
7. The composition of any of embodiments 1-6, wherein the NK cell subset is surface positive for a KIR, optionally KIR2DL2/3 and is negative or low for surface expression of Nkp30 and/or Nkp46.
8. The composition of any of embodiments 1-7, wherein the NK cell subset is surface positive for NKG2C.
9. The composition of any of embodiments 1-8, comprising from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells.
10. The composition of any of embodiments 1-9, wherein the volume of the composition is at least or at least about 10 mL, 50 mL, 100 mL, 200 mL, 300 mL, 400 mL or 500 mL and/or the composition comprises between 1 × 10⁵ and 1 × 10⁸cells/mL.
11. The composition of any of embodiments 1-10, further comprising a pharmaceutically acceptable carrier.
12. The composition of any of embodiments 1-11, further comprising a cyroprotectant.
13. The composition of any of embodiments 1-12, wherein the NK cell subset are primary NK cells obtained from a subject.
14. The composition of embodiment 13, wherein the subject is human.
15. A method for enriching NK cells, comprising:
   (a) isolating NK cells or a subset thereof from a subject identified as having the CD16 158V+ NK cell genotype; and
   (b) culturing the isolated NK cells or subset thereof under conditions to expand the cells, thereby enriching NK cells or the subset thereof from the subject.
16. The method of embodiment 15, comprising, prior to step (a), screening subjects for the presence of the CD16 158V+ NK cell genotype.
17. The method of embodiment 16, wherein the NK cells or subset thereof are isolated from a population of cells comprising lymphocytes.
18. The method of embodiment 17, wherein the population of cells comprises peripheral blood mononuclear cells (PBMC) sample, an apheresis product, or a leukapheresis product.
19. The method of any of embodiments 15-18, wherein an NK cell subset is isolated from the subject, the NK cell subset comprising or enriched for cells that do not express FcRy chain (CD16 158V+/g-).
20. The method of embodiment 19, wherein the NK cell subset is surface positive for a KIR, optionally KIR2DL2/3.
21. The method of any of embodiments 15-20, wherein the NK cell subset is negative or low for surface expression of Nkp30 and/or Nkp46.
22. The method of any of embodiments 15-21, wherein the NK cell subset is surface positive for a KIR, optionally KIR2DL2/3 and is negative or low for surface expression of Nkp30 and/or Nkp46.
23. The method of any of embodiments 15-22, wherein the NK cells or subset thereof is surface positive for NKG2C.
24. The method of any of embodiments 15-23, wherein the cells are cultured in the presence of feeder cells or in the presence of one or more cytokine, optionally IL-2, IL-15 and/or IL-7.
25. The method of any of embodiments 15-24, wherein the NK cells are cultured for a period of time to achieve expansion of the cells by at least 5-fold, 10-fold, 20-fold, 30-fold, 40-fold, 50-fold, 100-fold, 200-fold, 300-fold, 400-fold, 500-fold or more compared to the number of isolated NK cells prior to the culturing.
26. The method of any of embodiments 15-25, wherein the isolated NK cells are cultured for 7 to 28 days, optionally about 14 days, 15 days, 16 days, 17 days or 18 days, 19 days, 20 days, or 21 days.
27. The method of any of embodiments 15-26, wherein the isolated cells are cultured to achieve a therapeutically effective amount.
28. The method of any of embodiments 15-27, wherein the isolated cells are cultured to achieve a number of enriched NK cells from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells.
29. The method of any of embodiments 15-28, further comprising HLA typing the isolated or enriched cells.
30. The method of any of embodiments 15-29, further comprising cryopreserving the cells and/or formulating the cells in the presence of a cryoprotectant.
31. The method of any of embodiments 15-30, comprising administering IL-12, IL-15, IL-18, IL-2 and/or CCL5 to the subject prior to isolating the NK cells or subset thereof.
32. The method of any of embodiments 15-31, wherein the subject is human.
33. The method of any of embodiments 15-31, wherein the subject is healthy.
34. A composition, comprising enriched NK cells produced by the method of any of embodiments 15-33.
35. The composition of embodiment 34, comprising a pharmaceutically acceptable excipient.
36. The composition of embodiment 34 or embodiment 35, comprising a cyroprotectant.
37. The composition of any of embodiments 1-14 and 34-36 that is sterile.
38. A kit comprising the composition of any of embodiments 1-14 and 34-37 and an additional agent for treatment of a disease.
39. The kit of embodiment 38, further comprising instructions for administering the composition and additional agent for treating a disease or condition.
40. A kit comprising the composition of any of embodiments 1-14 and 34-37 and instructions for administering the composition in a combination therapy with an additional agent for treatment of a disease.
41. The kit of any of embodiments 38-40, wherein the additional agent is an antibody or an Fc-fusion protein.
42. The kit of embodiment 41, wherein the antibody recognizes or specifically binds a tumor associated antigen.
43. The kit of embodiment 42, wherein the tumor associated antigen is CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin.
44. The kit of any one of embodiments 41-43, wherein the antibody is a full length antibody and/or comprises an Fc domain.
45. A method of treating a disease or condition comprising administering the composition of any of embodiments 1-14 and 34-37 to an individual in need thereof.
46. The method of embodiment 45, wherein the administering comprises administration of from or from about 1 × 10⁸ to 1 × 10¹⁰ cells/m² to the individual or from or from about 1 × 10⁶ to 1 × 10¹⁰ NK cells/kg.
47. The method of embodiment 45 or embodiment 46 further comprising administering an additional agent.
48. The method of embodiment 47, wherein the additional agent is an antibody or an Fc-fusion protein.
49. The method of embodiment 48 wherein the antibody recognizes a tumor associated antigen.
50. The method of embodiment 48 or embodiment 49, wherein the antibody recognizes or specifically binds CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin.
51. The method of any one of embodiments 48-50, wherein the antibody comprises an Fc domain and/or is a full-length antibody.
52. The method of any one of embodiments 47-51, wherein the additional agent and the composition are administered sequentially.
53. The method of embodiment 52, wherein the additional agent is administered prior to administration of the composition.
54. The method of any one of embodiments 47-53, where the additional agent and the composition are administered simultaneously
55. The method of any one of embodiments 45-54, wherein the disease or condition is selected from the group consisting of an inflammatory condition, an infection, and cancer.
56. The method of embodiment 55, wherein the infection is a viral infection or a bacterial infection.
57. The method of embodiment 55, wherein the disease or condition is a cancer and the cancer is a leukemia or lymphoma.
58. The method of embodiment 55, wherein the disease or condition is a cancer and the cancer comprises a solid tumor.
59. The method of any one of embodiments 45-58, wherein the individual is a human.
60. The method of any one of embodiments 45-59, wherein the NK cells in the composition is allogenic to the individual.
61. The method of any one of embodiments 45-59, wherein the NK cells in the composition is autologous to the subject.

### VI. EXAMPLES

The following examples are included for illustrative purposes only and are not intended to limit the scope of the invention.

### Example 1: Isolation and Enrichment of g-158V + NK cell Subset from a Human Donor

To identify a subject carrying the V158+ polymorphism, DNA is extracted from peripheral blood of samples derived from healthy donors. Genomic DNA is extracted from the blood samples using the Wizard genomic DNA purification kit (Promega, Madison, WI). Genotyping for CD16 158V+ (V158V polymorphism) is carried out substantially as previously described, e.g. Cartron et al. (2002) Blood, 99:754-758; Koene et al. (1997) Blood, 90:1109-1114) using a nested PCR followed by allele-specific restriction enzyme digestion.

Briefly, the first PCR uses specific primers 5'-ATATTTACAGAATGGCACAGG-3' (SEQ ID NO:1) and 5'-GACTTGGTACCCAGGTTGAA-3') (SEQ ID NO:2) to amplify a 1.2 kilobase fragment containing the polymorphic site. The initial PCR assay is performed with approximately 1.25 µg genomic DNA, 200 ng of each primer, 200 µM of each deoxyribonucleoside triphosphate (dNTP) and 1 U Taq DNA polymerase. This first PCR is carried out for 10 minutes at 95°C for 35 cycles (each including steps at 95°C for 1 minute, 57°C for 1.5 minutes, and 72°C for 1.5 minutes), and 8 minutes at 72°C to achieve complete extension. The second PCR uses primers 5'-ATCAGATTCGATCCTACTTCTGCAGGGGGCAT-3' (SEQ ID NO:3) and 5'-ACGTGCTGAGCTTGAGTGATGGTGATGTTCAC-3' (SEQ ID NO:4), thereby amplifying a 94 base pair (bp) fragment and creating an N1aIII restriction site only in the 158V+ allele. This nested PCR is performed with 1 µL of the amplified DNA, 150 ng of each primer, 200 µM of each dNTP, and 1 U of Taq DNA polymerase. The cycle includes 5 minutes at 95°C, then 35 cycles (each including steps at 95°C for 1 minute, 64°C for 1 minute, and 72°C for 1 minute), and 9.5 minutes at 72°C to complete extension.

The amplified DNA (10 µL) is then digested with 10 U N1aIII at 37°C for 12 hours and separated by electrophoresis on 8% polyacrylamide gel. After staining with ethidium bromide, DNA bands are visualized under UV light. For homozygous 158F donors, only one undigested band (94 bp) is visible. Three bands (94 bp, 61 bp, and 33 bp) are seen in heterozygous individuals, whereas for homozygous 158V+ patients only 2 digested bands (61 bp and 33 bp) are obtained.

Peripheral blood mononuclear cells (PBMCs) from donors that are homozygous for 158V+ are obtained and surface markers of PBMCs are stained using antibodies specific to CD56 (N901), CD3 (UCHT1), CD14 (HCD14) and CD19 (HIB19) followed by fluorescence-activated cell sorting by gating of cells having the phenotype CD56^{dim}CD3⁻CD14⁻CD19⁻ and being surface positive for a KIR, either KIR2DL1 (CD158a; HP-MA4), KIR2DL2/3 (CD158b; CH-L) or KIR3DL1 (CD158e; DX9), and/or surface negative for an NCR, either Nkp46 (CD335; 9E2) or NKp30 (CD337; p30-15), thereby excluding non-NK cells and enriching for FcRy-deficient (g⁻NK) from freshly isolated PBMCs to about 70% or greater purity as described in Hwang et al. (2012) Int. Immunol., 24:793-802. Antibodies are obtained from commercial providers, such as Beckman Coulter (CA, USA), BD Biosciences (CA, USA), Biolegend (CA, USA) or eBiosciences (CA, USA).

For expansion, resulting enriched cells are plated by limiting dilution in NK cloning medium [RPMI1640 (Invitrogen, NY, USA) supplemented with 5% pooled human AB serum (Cellgro, VA, USA), 10% fetal bovine serum (Hyclone, UT, USA), 1 µg/ml PHA (Roche, IN, USA), 100U ml⁻¹ IL-2, and lOng ml⁻¹ IL-15 (Peprotech, NJ, USA)] along with PHA-stimulated (1 µg ml⁻¹ for 1h) allogeneic PBMCs and RPMI 8866 cells that had been treated with mitomycin C (Sigma-Aldrich, MO, USA) for 2h at 37°C. Feeder cells are added at 25,000 per well every 10-14 days for up to 2 months.

To assess antibody-directed activity of enriched CD16 158V+/g-NK cells, approximately multiple myeloma MM1S target cells are labeled with 100 µCi of ⁵¹Cr for 1 hour at 37° C., washed twice with PBS, and are resuspended in RPMI medium. A total of 3×10⁴ target cells in 100 µl RPMI medium are placed in triplicates into V-bottomed 96-well plates in the presence or absence of 10 µg/mL anti-CD38 antibody daratumumab. The tumor cells are incubated for about 20 minutes at room temperature and then are washed twice to remove antibody. The cells are resuspended in 100 µL medium to which is added an equal volume of CD16 158V+/g-NK cells at various effector:target ratios from 1:3 to 10:1 and cells are incubated for 4 hours. Aliquots of supernatants are counted using a Packard Cobra Auto-Gamma 5000 Series Counting System (Meridien, Conn., USA). The percentage specific ⁵¹Cr release is calculated according to the formula: percent specific release=[(experimental release - spontaneous release)/(maximum release-spontaneous release)]×100. As a control, experiments are carried out with CD56+ conventional NK cells or in the absence of anti-CD38 antibody daratumumab. The results show that CD16 158V+/g-NK cells exhibit ADCC activity in the presence of anti-CD38-coated tumor cells, which is greater than ADCC activity of the conventional NK cells.

Similar experiments are performed with rituximab-coated CD20+ B cell lymphoma cells. The results show that CD16 158V+/g-NK cells exhibit greater ability to mediate ADCC when co-cultured with rituximab-coated EBV-transformed B-cell lymphoma cells than conventional NK cells or controls.

### Example 2: Assessment of antibody-dependent cellular cytotoxicity (ADCC) of NK cell subsets

158V is a genetic polymorphism of CD16 where the amino acid valine (V) is present at the 158th amino acid position of the protein instead of the more common phenylalanine (F) (Koene et al., 1997, Blood 90:1109-14). This leads to greater expression and antibody affinity of CD16, which results in enhanced ADCC by CD16 158V+ NK-cells (Hatjiharissi et al., 2007, Blood 110:2561-4). Specifically, 158 V/V and 158 V/F donors kill ARH-77 and Daudi cells via ADCC far better than 158 F/F donors with 158 V/V donors performing the best (Hatjiharissi et al., 2007).

Forty (40) CMV-seropositive donors from BloodWorks NW were screened to determine the 12 donors with the highest proportion of g-NK. These donors g-NK cell were enriched from PBMCs through magnetic bead separation (CD3-/CD57+). Bulk NK-cells from conventional donors also were enriched from PBMCs from donors who had no g-NK through magnetic bead separation (CD3-/CD57+). The proportion of g-NK cells was determined using flow cytometry using fluorescent antibodies for the following extracellular markers: CD45, 7-AAD, CD3, CD56, CD16, CD57, CD7, and CD161. After incubation with antibodies for 20 minutes at room temperature in the dark, the cells were washed with PBS and the number of 7-AAD^{neg}/CD45^{pos} leukocytes was quantified by flow cytometry. The cells were washed, fixed with 4% paraformaldehyde, and washed and resuspended in 0.2% saponin to permeabilize the cell membrane. Then, fluorescently-conjugated anti-FceRI antibody was added. Following an additional 15-minute incubation at room temperature in the dark, the cells were analyzed flow cytometrically and the percentage of g-NK was assessed as the percentage of CD45^{pos}/7-AAD^{neg}/CD3^{neg}/CD56^{pos}/FceRI^{neg}lymphocytes.

The enriched cells were tested for ADCC against the following tumor/antibody combinations: 1) SW-480/cetuximab; 2) SKOV3/trastuzumab; 3) SKOV3/cetuximab; 4) MM.1S/daratumumab; and 5) MM.1S/elotuzumab.

For the ADCC cytotoxicity assay against the ovarian cancer cell line SKOV3 (ATCC; Manassas, VA, USA) as targets, NK-cells were co-cultured with CD71-labeled SKOV3 target cells (1.0 × 10⁴ cells) at 0.5:1, 1:1, 2.5:1, and 5:1 NK-cell: target cell ratios in a final volume of 2.2 mL of target cell-specific media (Bigley et al., 2014, Brain Behav Immuno., 39:160-71). The media used for the ADCC assay was 10% FBS-supplemented McCoy's 5A media with 1 µg/mL Trastuzumab (anti-HER2) or 5 µg/mL Cetuximab (anti-EGFR). In each case, basal cytotoxicity was also measured without the treating antibody present. Target cell only tubes were used to control for spontaneous cell death (less than 10% for all assays). After a 4h incubation at 37° C, the cells were washed and stained with anti-CD3 and CD56 antibodies to quantify the number of NK-cells in the tube. After a final wash, propidium iodide (PI) was added and the number of NK-cells, live target cells, and dead target cells were resolved using 4-color flow cytometry (Bigley et al., 2018, J Appl Physiol, 126:842-853).

For the ADCC cytotoxicity assay against the multiple myeloma cell line MM.1S (ATCC; Manassas, VA, USA) as targets. NK-cells were co-cultured with CD71-labeled MM.1S target cells (1.0 × 10⁴ cells) at 0.5:1, 1:1, 2.5:1, and 5:1 NK-cell: target cell ratios in a final volume of 2.2 mL of target cell-specific media. The media used for the ADCC assay was 10% FBS-supplemented RPMI-1640 media with 1 µg/mL Daratumumab (anti-CD38) or 1 µg/mL Elotuzumab (anti-CD319). In each case, basal cytotoxicity was also measured without the treating antibody present. Target cell only tubes were used to control for spontaneous cell death (less than 10% for all assays). After a 4h incubation at 37° C, the cells were washed and stained with anti-CD3 and CD56 antibodies to quantify the number of NK-cells in the tube. After a final wash, propidium iodide (PI) was added and the number of NK-cells, live target cells, and dead target cells were resolved using 4-color flow cytometry (Bigley et al., 2018).

For the ADCC cytotoxicity assay against the CRC cell line SW-480 (ATCC; Manassas, VA, USA) as targets, NK-cells were co-cultured with CD71-labeled SW-480 target cells (1.0 × 10⁴ cells) at 0.5:1, 1:1, 2.5:1, and 5:1 NK-cell: target cell ratios in a final volume of 2.2 mL of target cell-specific media. The media used for the ADCC assay was 10% FBS-supplemented Leibovitz's L-15 Medium with 5 µg/mL Cetuximab (anti-EGFR). Basal cytotoxicity was also measured without the Cetuximab present. Target cell only tubes were used to control for spontaneous cell death (less than 10% for all assays). After a 4h incubation at 37° C, the cells were washed and stained with anti-CD3 and CD56 antibodies to quantify the number of NK-cells in the tube. After a final wash, propidium iodide (PI) was added and the number of NK-cells, live target cells, and dead target cells were resolved using 4-color flow cytometry (Bigley et al., 2018).

As shown in **FIG. 1****,** the ADCC of the g-NK was compared to conventional NK-cells (c-NK) from donors who had no g-NK (n=4). Of these 12 donors, 5 'super donors' stood out for their consistently high ADCC values.

The donors were then assessed to determine which subgroup each donor belonged to with regards to the 158V polymorphism of CD16 (V/V, V/F, and F/F). The expected distribution was 35% V/V, 25% V/F, and 40% F/F (Hatjiharissi et al., 2007; Somboonyosdech et al., 2012, Asian Biomedicine, 6:883-89], thus any deviation from this expectation would be consistent with a finding that the 158V polymorphism may play a role in the high ADCC seen with these donors. For the polymorphism testing, frozen NK-cells were thawed and washed with PBS and centrifuged at 100 g. Following the wash step, the supernatant was decanted and the NK-cells were resuspended in 10 mL for cell counting by flow cytometry. Briefly, 50 µL of the cell suspension were collected into a flow tube and stained with 2 µL of a fluorescent antibody for CD45 (to discern leukocytes from residual red blood cells) and 2 µL of 7-AAD (a viability dye). Following a 10 minute incubation at room temperature in the dark, the cells were diluted with 500 µL of PBS and the number of 7-AAD^{neg}/CD45^{pos} leukocytes was quantified by flow cytometry. Following the cell count, a magnetic bead separation was conducted to isolate a population of CD16^{pos} NK-cells. For this protocol, the magnetic bead separation was performed using Miltenyi MACS^{™} CD16 Microbeads as per manufacturer's instructions. Following the magnetic bead separation, 10X Genomics single cell RNA sequencing was used to determine which CD16 polymorphic group the donors belong to (V/V, V/F, or F/F). As shown in **FIG. 1****,** 158V (V/V or V/F) donors have the highest ADCC.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### VII. FURTHER EXEMPLARY EMBODIMENTS

1. A composition comprising a natural killer (NK) cell subset surface positive for CD16 158V+, wherein at least about 50%, at least about 60%, at least about 70%, at least about 80%, or at least about 90% of the cells in the composition comprise the CD16 158V+ NK cell subset.
2. The composition of embodiment 1, wherein the CD16 158V+ NK cell subset does not express FcRy chain (CD16 158V+/g-), is surface positive for a KIR and/or is surface negative or low for Nkp30 and/or Nkp46.
3. A composition comprising a natural killer (NK) cell subset surface positive for CD16 158V+ and deficient in expression of FcRy chain (CD16 158V+/g-), wherein at least about 50%, at least about 60%, at least about 70%, at least about 80%, or at least about 90% of the cells in the composition comprise the CD16 158V+/g- NK cell subset.
4. The composition of any of embodiments 1-3, wherein CD16 158V+ comprises the sequence set forth in SEQ ID NO: 11 or a sequence that exhibits at least 85%, 90% or 95% sequence identity to SEQ ID NO:11 and comprises the 158V+ polymorphism.
5. The composition of any of embodiments 1-4, wherein the NK cell subset is surface positive for a KIR, optionally KIR2DL2/3.
6. The composition of any of embodiments 1-5, wherein the NK cell subset is negative or low for surface expression of Nkp30 and/or Nkp46.
7. The composition of any of embodiments 1-6, wherein the NK cell subset is surface positive for a KIR, optionally KIR2DL2/3 and is negative or low for surface expression of Nkp30 and/or Nkp46.
8. The composition of any of embodiments 1-7, wherein the NK cell subset is surface positive for NKG2C.
9. The composition of any of embodiments 1-8, comprising from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells.
10. The composition of any of embodiments 1-9, wherein the volume of the composition is at least or at least about 10 mL, 50 mL, 100 mL, 200 mL, 300 mL, 400 mL or 500 mL and/or the composition comprises between 1 × 10⁵ and 1 × 10⁸cells/mL.
11. The composition of any of embodiments 1-10, further comprising a pharmaceutically acceptable carrier.
12. The composition of any of embodiments 1-11, further comprising a cyroprotectant.
13. The composition of any of embodiments 1-12, wherein the NK cell subset are primary NK cells obtained from a subject.
14. The composition of embodiment 13, wherein the subject is human.
15. A method for enriching NK cells, comprising:
   (a) isolating NK cells or a subset thereof from a subject identified as having the CD16 158V+ NK cell genotype; and
   (b) culturing the isolated NK cells or subset thereof under conditions to expand the cells, thereby enriching NK cells or the subset thereof from the subject.
16. The method of embodiment 15, comprising, prior to step (a), screening subjects for the presence of the CD16 158V+ NK cell genotype.
17. The method of embodiment 15 or embodiment 16, wherein the NK cells or subset thereof are isolated from a population of cells comprising lymphocytes.
18. The method of embodiment 17, wherein the population of cells comprises peripheral blood mononuclear cells (PBMC) sample, an apheresis product, or a leukapheresis product.
19. The method of any of embodiments 15-18, wherein an NK cell subset is isolated from the subject, the NK cell subset comprising or enriched for cells that do not express FcRy chain (CD16 158V+/g-).
20. The method of embodiment 19, wherein the NK cell subset is surface positive for a KIR, optionally KIR2DL2/3.
21. The method of any of embodiments 15-20, wherein the NK cell subset is negative or low for surface expression of Nkp30 and/or Nkp46.
22. The method of any of embodiments 15-21, wherein the NK cell subset is surface positive for a KIR, optionally KIR2DL2/3 and is negative or low for surface expression of Nkp30 and/or Nkp46.
23. The method of any of embodiments 15-22, wherein the NK cells or subset thereof is surface positive for NKG2C.
24. The method of any of embodiments 15-23, wherein the cells are cultured in the presence of feeder cells or in the presence of one or more cytokine, optionally IL-2, IL-15 and/or IL-7.
25. The method of any of embodiments 15-24, wherein the NK cells are cultured for a period of time to achieve expansion of the cells by at least 5-fold, 10-fold, 20-fold, 30-fold, 40-fold, 50-fold, 100-fold, 200-fold, 300-fold, 400-fold, 500-fold or more compared to the number of isolated NK cells prior to the culturing.
26. The method of any of embodiments 15-25, wherein the isolated NK cells are cultured for 7 to 28 days, optionally about 14 days, 15 days, 16 days, 17 days or 18 days, 19 days, 20 days, or 21 days.
27. The method of any of embodiments 15-26, wherein the isolated cells are cultured to achieve a therapeutically effective amount.
28. The method of any of embodiments 15-27, wherein the isolated cells are cultured to achieve a number of enriched NK cells from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells.
29. The method of any of embodiments 15-28, further comprising HLA typing the isolated or enriched cells.
30. The method of any of embodiments 15-29, further comprising cryopreserving the cells and/or formulating the cells in the presence of a cryoprotectant.
31. The method of any of embodiments 15-30, comprising administering IL-12, IL-15, IL-18, IL-2 and/or CCL5 to the subject prior to isolating the NK cells or subset thereof.
32. The method of any of embodiments 15-31, wherein the subject is human.
33. The method of any of embodiments 15-31, wherein the subject is healthy.
34. A composition, comprising enriched NK cells produced by the method of any of embodiments 1-33.
35. The composition of embodiment 34, comprising a pharmaceutically acceptable excipient.
36. The composition of embodiment 34 or embodiment 35, comprising a cyroprotectant.
37. The composition of any of embodiments 1-14 and 34-36 that is sterile.
38. A kit comprising the composition of any of embodiments 1-14 and 34-37 and an additional agent for treatment of a disease.
39. The kit of embodiment 38, further comprising instructions for administering the composition and additional agent for treating a disease or condition.
40. A kit comprising the composition of any of embodiments 1-14 and 34-37 and instructions for administering the composition in a combination therapy with an additional agent for treatment of a disease.
41. The kit of any of embodiments 38-40, wherein the additional agent is an antibody or an Fc-fusion protein.
42. The kit of embodiment 41, wherein the antibody recognizes or specifically binds a tumor associated antigen.
43. The kit of embodiment 41 or embodiment 42, wherein the antibody recognizes or binds CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin.
44. The kit of any one of embodiments 41-43, wherein the antibody is a full length antibody and/or comprises an Fc domain.
45. A method of treating a disease or condition comprising administering the composition of any of embodiments 1-14 and 34-37 to an individual in need thereof.
46. The method of embodiment 45, wherein the administering comprises administration of from or from about 1 × 10⁸ to 1 × 10¹⁰ cells/m² to the individual or from or from about 1 × 10⁶ to 1 × 10¹⁰ NK cells/kg.
47. The method of embodiment 45 or embodiment 46 further comprising administering an additional agent.
48. The method of embodiment 47, wherein the additional agent is an antibody or an Fc-fusion protein.
49. The method of embodiment 48 wherein the antibody recognizes a tumor associated antigen.
50. The method of embodiment 48 or embodiment 49, wherein the antibody recognizes or specifically binds CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin.
51. The method of any one of embodiments 48-50, wherein the antibody comprises an Fc domain and/or is a full-length antibody.
52. The method of any one of embodiments 47-51, wherein the additional agent and the composition are administered sequentially.
53. The method of embodiment 52, wherein the additional agent is administered prior to administration of the composition.
54. The method of any one of embodiments 47-53, where the additional agent and the composition are administered simultaneously.
55. The method of any one of embodiments 45-54, wherein the disease or condition is selected from the group consisting of an inflammatory condition, an infection, and cancer.
56. The method of embodiment 55, wherein the disease or condition is an infection and the infection is a viral infection or a bacterial infection.
57. The method of embodiment 55, wherein the disease or condition is a cancer and the cancer is a leukemia or lymphoma.
58. The method of embodiment 55, wherein the disease or condition is a cancer and the cancer comprises a solid tumor.
59. The method of any one of embodiments 45-58, wherein the individual is a human.
60. The method of any one of embodiments 45-59, wherein the NK cells in the composition is allogenic to the individual.
61. The method of any one of embodiments 45-59, wherein the NK cells in the composition is autologous to the subject.

## Claims

1. A composition comprising a natural killer (NK) cell subset surface positive for CD16 158V+, wherein at least about 50%, at least about 60%, at least about 70%, at least about 80%, or at least about 90% of the cells in the composition comprise the CD16 158V+ NK cell subset.

2. The composition of claim 1, wherein the CD16 158V+ NK cell subset does not express FcRy chain (CD16 158V+/g-), is surface positive for a KIR and/or is surface negative or low for Nkp30 and/or Nkp46.

3. A composition comprising a natural killer (NK) cell subset surface positive for CD16 158V+ and deficient in expression of FcRγ chain (CD16 158V+/g-), wherein at least about 50%, at least about 60%, at least about 70%, at least about 80%, or at least about 90% of the cells in the composition comprise the CD16 158V+/g- NK cell subset.

4. The composition of any of claims 1-3, wherein
(a) the CD16 158V+ comprises the sequence set forth in SEQ ID NO: 11 or a sequence that exhibits at least 85%, 90% or 95% sequence identity to SEQ ID NO: 11 and comprises the 158V+ polymorphism; and/or
(b) the NK cell subset is surface positive for a KIR, optionally KIR2DL2/3; and/or
(c) the NK cell subset is negative or low for surface expression of Nkp30 and/or Nkp46; and/or
(d) the NK cell subset is surface positive for a KIR, optionally KIR2DL2/3 and is negative or low for surface expression of Nkp30 and/or Nkp46; and/or
(e) the NK cell subset is surface positive for NKG2C; and/or
(f) the composition comprises from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells; and/or
(g) volume of the composition is at least or at least about 10 mL, 50 mL, 100 mL, 200 mL, 300 mL, 400 mL or 500 mL and/or the composition comprises between 1 × 10⁵ and 1 × 10⁸cells/mL; and/or
(h) the composition further comprises a pharmaceutically acceptable carrier or a cyroprotectant; and/or
(i) the NK cell subset are primary NK cells obtained from a subject, optionally wherein the subject is human.

5. A method for enriching NK cells, comprising:
(a) providing NK cells or a subset thereof isolated from a subject identified as having the CD16 158V+ NK cell genotype; and
(b) culturing the isolated NK cells or subset thereof under conditions to expand the cells, thereby enriching NK cells or the subset thereof from the subject.

6. The method of claim 5, wherein:
(A) the method comprises, prior to step (a), screening subjects for the presence of the CD16 158V+ NK cell genotype; and/or
(B) the NK cells or subset thereof are isolated from a population of cells comprising lymphocytes, optionally wherein the population of cells comprises peripheral blood mononuclear cells (PBMC) sample, an apheresis product, or a leukapheresis product; and/or
(C) an NK cell subset has been isolated from the subject, the NK cell subset comprising or enriched for cells that do not express FcRy chain (CD16 158V+/g-), optionally wherein the NK cell subset is surface positive for a KIR, optionally KIR2DL2/3; and/or
(D) the NK cell subset is negative or low for surface expression of Nkp30 and/or Nkp46; and/or
(E) the NK cell subset is surface positive for a KIR, optionally KIR2DL2/3 and is negative or low for surface expression of Nkp30 and/or Nkp46; and/or
(F) the NK cells or subset thereof is surface positive for NKG2C; and/or
(G) the cells are cultured in the presence of feeder cells or in the presence of one or more cytokine, optionally IL-2, IL-15 and/or IL-7; and/or
(H) the NK cells are cultured for a period of time to achieve expansion of the cells by at least 5-fold, 10-fold, 20-fold, 30-fold, 40-fold, 50-fold, 100-fold, 200-fold, 300-fold, 400-fold, 500-fold or more compared to the number of isolated NK cells prior to the culturing; and/or
(I) the isolated NK cells are cultured for 7 to 28 days, optionally about 14 days, 15 days, 16 days, 17 days or 18 days, 19 days, 20 days, or 21 days; and/or
(J) the isolated cells are cultured to achieve a therapeutically effective amount; and/or
(K) the isolated cells are cultured to achieve a number of enriched NK cells from or from about 10⁵ to about 10¹² cells, from or from about 10⁵ to about 10⁸ cells, from or from about 10⁶ to about 10¹² cells, from or from about 10⁸ to about 10¹¹ cells, or from or from about 10⁹ to about 10¹⁰ cells; and/or
(L) the method further comprises HLA typing the isolated or enriched cells; and/or
(M) the method further comprises cryopreserving the cells and/or formulating the cells in the presence of a cryoprotectant; and/or
(N) the method comprises administering IL-12, IL-15, IL-18, IL-2 and/or CCL5 to the subject prior to isolating the NK cells or subset thereof; and/or
(O) the subject is human and/or healthy.

7. A composition, comprising enriched NK cells produced by the method of claims 5 or 6.

8. The composition of claim 7, comprising a pharmaceutically acceptable excipient and/or a cyroprotectant.

9. The composition of any of claims 1-4 and 7-8 that is sterile.

10. A kit comprising the composition of any of claims 1-4 and 7-8 and an additional agent for treatment of a disease.

11. The kit of claim 10, further comprising instructions for administering the composition and additional agent for treating a disease or condition.

12. A kit comprising the composition of any of claims 1-4 and 7-8 and instructions for administering the composition in a combination therapy with an additional agent for treatment of a disease.

13. The kit of any of claims 10-12, wherein the additional agent is an antibody or an Fc-fusion protein, optionally wherein
(a) the antibody recognizes or specifically binds a tumor associated antigen; and/or
(b) the antibody recognizes or binds CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin; and/or
(c) the antibody is a full length antibody and/or comprises an Fc domain.

14. The composition of any of claims 1-4 and 7-8 for use in treating a disase or condition in an individual in need thereof.

15. The composition for use according to claim 14, wherein
(A) the treating comprises administration of from or from about 1 × 10⁸ to 1 × 10¹⁰ cells/m² to the individual or from or from about 1 × 10⁶ to 1 × 10¹⁰ NK cells/kg to the subject; and/or
(B) the treating further comprises administering an additional agent, optionally wherein
(a) the additional agent is an antibody or an Fc-fusion protein, further optionally wherein
(i) the antibody recognizes a tumor associated antigen; and/or
(ii) the antibody recognizes or specifically binds CD19, CD20, CD22, CD30, CD33, CD37, CD38, CD40, CD52, CD56, CD70, CD74, CD140, EpCAM, CEA, gpA33, mesothelin, α-fetoprotein, Mucin, PDGFR-alpha, TAG-72, CAIX, PSMA, folate-binding protein, scatter factor receptor kinase, a ganglioside, cytokerain, frizzled receptor, VEGF, VEGFR, Integrin αVβ3, integrin α5β1, EGFR, EGFL7, ERBB2 (HER2), ERBB3, fibronectin, HGF, HER3, LOXL2, MET, IGF1R, IGLF2, EPHA3, FR-alpha, phosphatidylserine, Syndecan 1, SLAMF7 (CD319), TRAILR1, TRAILR2, RANKL, FAP, vimentin or tenascin; and/or
(iii) the antibody comprises an Fc domain and/or is a full-length antibody; and/or
(b) the additional agent and the composition are administered sequentially, optionally wherein the additional agent is administered prior to administration of the composition; and/or
(c) the additional agent and the composition are administered simultaneously; and/or
(C) the disease or condition is selected from the group consisting of an inflammatory condition, an infection, and cancer, optionally wherein the disease or condition is:
(a) an infection and the infection is a viral infection or a bacterial infection; or
(b) a cancer and the cancer is a leukemia, lymphoma or comprises a solid tumor; and/or
(D) the individual is a human; and/or
(E) the NK cells in the composition is allogenic or autologous to the individual.
